# EUROPEAN PATENT APPLICATION

(11) **EP 1 514 558 A1**
(43) Date of publication of application: **16.03.2005**
(21) Application number: 03400053.9
(22) Date of filing: 15.09.2003
(51) Int. Cl.: A61K 38/45, A61P 25/28, A61P 35/00, A61K 39/395, A61K 48/00

(54) **Use of MARKK or MARKK antagonists for the treatment of pathologies characterised by increased or reduced phosphorylation of MARK or tau protein**

(71) Applicant: Max-Planck-Gesellschaft zur Förderung der Wissenschaften e.V., 80539 München (DE)
(72) Inventor: Mandelkow, Eva-Maria, Dr., 22607 Hamburg (DE); Mandelkow, Eckhard, 22607 Hamburg (DE); Timm, Thomas, 22549 Hamburg (DE); Biernat, Jacek, 22869 Schenefeld (DE); Li, Xiaoyu, 21073 Hamburg (DE); Matenia, Dorthe, 24558 Henstedt-Ulzburg (DE); von Bergen, Martin, 21484 Gülzow (DE)
(74) Representative: UEXKÜLL & STOLBERG

(57) **Abstract**

This invention relates to the use of MARKK or MARKK antagonists for the preparation of a pharmaceutical composition for the treatment of pathologies characterised by increased or reduced phosphorylation of MARK or tau protein such as Alzheimer disease.

## Description

The present invention relates to the use of MARKK (microtubule affinity regulating kinase kinase) or MARKK antagonists for the preparation of a pharmaceutical composition for the treatment of conditions characterised by increased or reduced phosphorylation of MARK or tau protein such as Alzheimer disease.

Alzheimer's disease (AD) is the most common cause of dementia in the middle-aged and the elderly and is responsible for about 50% of all cases of senile dementia in North America and Western Europe (IQBAL, K. & GRUNDKE-IQBAL, I. 1997). AD is a neurodegenerative disease histopathologically characterised by two characteristic types of protein deposits in the brain. Deposits consisting of amyloid precursor protein (APP) can be found extracellularly, whereas the other protein deposit primarily manifests inside the neurons of the brain. These intracellular deposits are neurofibrillary tangles of paired helical filaments (PHF) consisting of the microtubule-associated protein tau as the major protein subunit. It has been found that the tau protein in these PHF is hyperphosphorylated.

The tau protein belongs to a class of microtubule-associated proteins (MAPs) expressed in mammalian brain that regulate the extensive dynamics and rearrangement of the microtubule networks in the cells. The phosphorylation state of MAPs is balanced by kinases and phosphatases and plays a pivotal role in the modulation of these events. There are two classes of phosphorylation sites in tau protein. The Serine-Proline (SP) or Threonine-Proline (TP) sites clustered before and after the repeat region of tau which can be phosphorylated by proline directed kinases as well as the serines within the KXGS motifs in the repeats phosphorylated by MARK (microtubule affinity regulating kinase), a kinase family related to PAR-1. This kinase is involved in establishing cell polarity, tau phosphorylation and phosphorylation of related MAPS (MAP2, MAP4) at serines within the 3-4 KXGS motifs in the microtubule-binding domain (S262, S293, S324 and S356 according to the numbering of htau40; GOEDERT, M. et al. 1988) Phosphorylation causes detachment of tau and destabilisation of microtubules, which represent the basis for axonal transport (DREWES, G. et al. 1997, STAMER, K. et al. 2002).

Expression of a family of MARKs can disrupt microtubules in cells and cause disassembly. These MARKS are homologous to the KIN1/PAR-1/Snf1 kinase family of the CaMK group II (HANKS, S.K. & Hunter, T. 1995). Their common feature is the involvement in generating cell polarity, e.g. in yeast budding, partitioning of the C. elegans zygote, or embryonic axis formation in Drosophila (Guo, S. & KEMPHUES, K.J. 1995; SHULMAN, J.M. et al., 2000; TOMANCAK, P. et al. 2000). In mammalian polarised cells such as epithelia or neurons, MARK/PAR-1 plays a role in the asymmetric organisation of the cell membrane and cell components (BÖHM, H. et al. 1997; BIERNAT, J. et al. 2002).

The mechanisms of tau protein phosphorylation by activation of MARK by upstream factors is presently unknown. MARKs from brain contain a double phosphorylated peptide in the activation loop of the catalytic domain (DREWES, G. et al. 1997), T215/S219 in MARK1 or T208/S212 in MARK2, T211/S215 in MARK3 and T214/S218 in MARK4. Many kinases are activated by phosphorylation of a threonine in the activation loop (T208 in MARK2, by homology with PKA) which forms an ion pair with an arginine and a lysine (R174 and K96 in MARK2) that are important for fixing the loop. Some kinases require dual phosphorylation at the threonine and a downstream tyrosine, e.g. MAP kinases which are activated by a MAPKK (or MEK), which in turn becomes activated by upstream extracellular signals (CHEN, Z. et al. 2001).

The kinase(s) responsible for the activation of MARK has not been identified. Since the activity of the tau protein is controlled by these kinases and the tau protein itself is involved in generating AD, it would be highly desirable to regulate MARK activity via the natural kinase responsible for MARK phosphorylation or an inhibitor thereof. The present invention specifically addresses this problem.

The above problem is now solved by the use of MARKK (microtubule affinity regulating kinase kinase) for the preparation of a pharmaceutical composition for the treatment of pathologies characterised by increased or reduced phosphorylation of MARK or tau protein. According to a preferred embodiment, the use comprises the preparation of a pharmaceutical composition for the treatment of Alzheimer's disease.

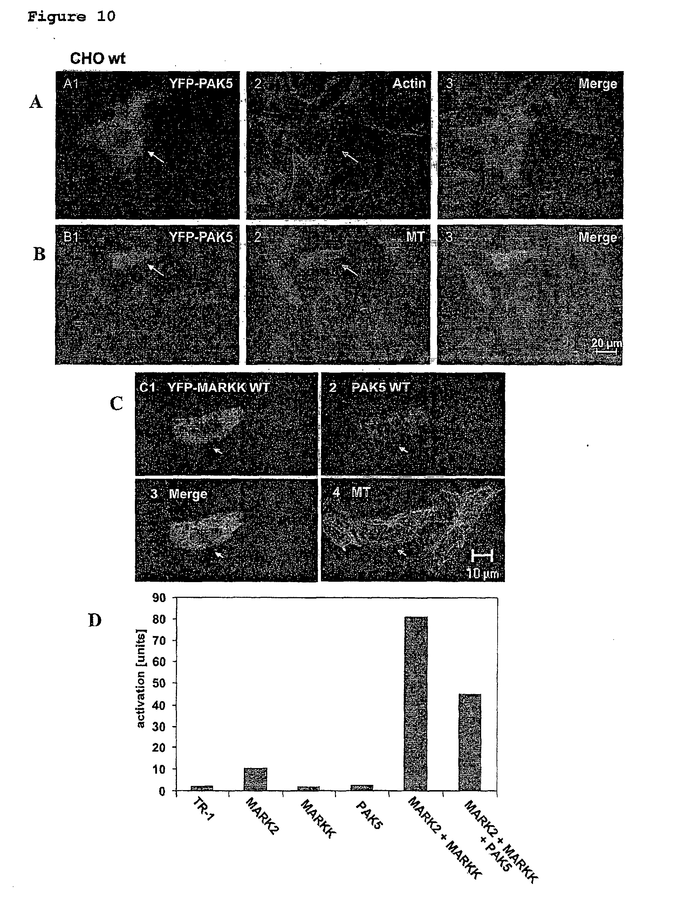

Studying the activity of MARK the present inventors were for the first time able to identify and purify a kinase responsible for MARK phosphorylation. The enzyme thus identified, MARKK (see Examples 1, 2 and 3 and Figure 10B), is highly homologous to the previously known kinase TAO-1 (HUTCHISON, M. et al. 1998) and belongs to the Ste20-family of kinases. This family comprises the p21-activated protein kinases (PAKs, with an N-terminal p21-binding domain and a C-terminal kinase domain) and the germinal centre kinases (GCKs; N-terminal kinase domain, no p21-binding domain). About 31 Ste20-related kinases are known (2 PAK-, 8 GCK-subfamilies, MANNING, G. et al. 2002).

These kinases have various effects including the regulation of apoptosis and the rearrangement of the cytoskeleton, and some are known to play a role in the activation of MAP kinases. Regulation of Ste20 kinases is achieved via the Ras-family of GTPases, additional protein kinases, adapter proteins coupled to cytokine receptors, and via dimerisation or association with inhibitors combined with autophosphorylation after stimulation (DAN, I. et al. 2001). MARKK/TAO-1 is part of the kinase subfamily GCK-VIII, together with the kinases PSK/TAO-2 (CHEN, Z. et al. 1999) and JIK/TAO-3 (TASSI, E. et al. 1999).

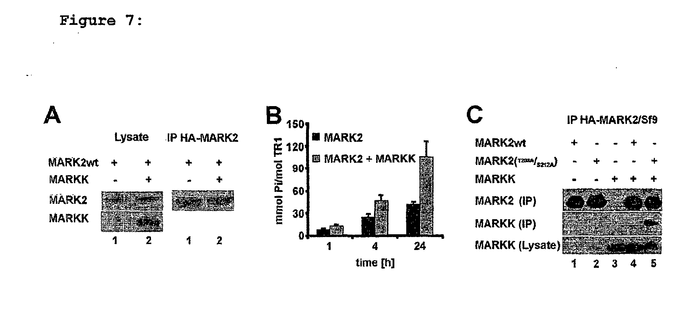

The present application for the first time shows that the activity of MARKK enhances microtubule dynamics in cells via activation of MARK which leads to phosphorylation and detachment of tau or equivalent MAPs from microtubules (see Example 4). In CHO cells, overexpression of MARKK triggers a cascade of activation of MARK and phosphorylation of MAPs at KXGS motifs, leading to increased microtubule dynamics, subsequent breakdown of microtubules and cell death (see Figure 7). In neuronal cells (PC12), MARKK and MARK increase their activity upon differentiation by NGF (nerve growth factor). This leads to phosphorylation of tau at KXGS motifs, increased microtubule dynamics, and enables initiation of neurites (BIERNAT, J. et al. 2002).

According to a preferred embodiment of the invention MARKK or MARKK antagonists are used for the preparation of a pharmaceutical composition for the treatment of pathologies characterised by increased or reduced phosphorylation of MARK.

According to an alternative embodiment of the invention MARKK or MARKK antagonists are used for the preparation of a pharmaceutical composition for the treatment of pathologies characterised by increased or reduced phosphorylation of tau protein at KXGS sites.

In the past it has been shown in cell models and animal models that upon phosphorylation at the KXGS motifs tau protein detaches from microtubuli (MT), leading to MT breakdown, accumulation and aggregation of tau in the cell body resulting eventually in cell death (for a review see DREWES, G. et al. 1998; VOGELSBERG-RAGAGLIA, V. et al. 2000; LEWIS, J. et al. 2000; STAMER et al. 2002). Increased phosphorylation of tau protein by loss of affinity towards the MT results in less tau protein bound to MTs compared to healthy differentiated cells.

Further, it was surprisingly shown by the inventors that degeneration of neurons is not only caused by hyperphosphorylation of the KXGS sites of tau protein and subsequent detachment of the protein from microtubules leading to the disassembly of these structures and eventually to the apoptosis of these cells, but also by hypophosphorylation of KXGS sites leading to increased binding of tau to the microtubule surface in tau overexpressing neurons with the consequence of overstabilizing microtubules and inhibiting anterograde transport of vesicles and organelles towards the synapse. This eventually results also in neuronal degeneration (STAMER, et al. 2002) by impairment of nourishment of the synapses.

It is thus clear that it is necessary to adjust and maintain a balance between hyperphosphorylated tau protein on one side and hypophosphorylated tau on the other side at the KXGS motifs as can be found in healthy differentiated cells to effectively treat AD and related diseases.

The present invention is thus amongst others based on the observation that in the development of a pathology characterised by impaired phosphorylation of tau protein, such as AD, different phases can be distinguished. In an early phase an excess of hypophosphorylated tau is bound to MT hindering cellular transport processes along the MT which eventually may lead to apoptosis of the cell. The cells respond to the impaired transport by upregulation of the activity of MARKK to provide the active kinase responsible for the phosphorylation of the bound tau proteins, which detaches from the MT upon phosphorylation. In a later phase the regulation of the MARKK itself becomes impaired resulting in overactivation of MARKK and MARK and hyperphosphorylated tau protein, that detaches from the MT resulting in microtubule breakdown and subsequent cell death.

Activity of MARK and the activating Kinase MARKK can be measured by the *in vitro* phosphorylation of substrate peptides in the presence of radioactive labelled ATP (DREWES, G. et al. 1995; TIMM, T. et al. 2003). The degree of phosphorylation of tau can be quantified in extracts of AD brains by immunochemical analysis (Elisa, Western blot, immunoprecipitation) using antibodies (such as 12E8, see SEUBERT, P. et al. 1995) specific for the phosphorylated KXGS motifs of Serine262 and Serine356 of repeat one and three of tau. In brain sections this antibody can further be used for immunohistochemistry.

Analysis of the activity of MARK can be carried out using the antibody (BIERNAT, J. et al. 2002; TIMM, T et al. 2003) against the phosphorylated T210 in MARK1, T208 in MARK2, T211 in MARK3, T214 in MARK4 in the regulatory loop with the same methods described above.

Increased or reduced phosphorylation of MARK and MAPs (tau, MAP2, MAP4) at KXGS motifs may be present in all cells containing a microtubule network.

In an equally preferred embodiment of the invention the increased or reduced phosphorylation of MARK and tau and MAP2 at KXGS motifs is present in neurons of a mammal.

In a particularly preferred embodiment, said mammal is a human.

In another aspect of the present invention the pathology characterised by increased or reduced phosphorylation of MARK and/or tau protein that may be treated by the use of MARKK antagonists and MARKK is Alzheimer's disease.

MARKK antagonists could for example be tested in cell culture or animal models of AD. Antagonists that reduce MARK phosphorylation and are well tolerated can be used to treat AD in cases, wherein diagnosis of the disease has revealed that tau hyperphosphorylation is responsible for tau aggregation and disease onset.

In a further preferred embodiment of the invention MARKK or MARKK antagonists are used for the preparation of a pharmaceutical composition for the treatment of pathologies wherein the treatment is continued until more than 20% of the total tau protein is bound to microtubules. Again the effect of the drug could be assayed in AD resembling cell culture models and animal models.

The inventors have shown that binding of less than 20 % of total tau proteins to MT leads to MT-destabilisation. Detachment of 80% of total tau from the MTs causes breakdown of the MTs tracks for vesicle and organelle transport. Since it has been shown that cell polarisation requires an asymmetric distribution of cell components whose transport involves microtubules, this degradation of microtubules results in axonal degeneration and loss of neuronal polarity and eventually apoptosis of the cell (DREWES, G. et al. 1997; EBNETH, A. et al. 1999; BIERNAT, J et al. 2002).

In further preferred embodiments, other pathologies exhibiting increased phosphorylation of tau protein resulting in less than 20 % of total tau binding to MTs that can be treated by use of MARKK antagonists are selected from the group of Tauopathies consisting of CBD (Cortical Basal Disease), PSP (Progressive Supra Nuclear Palsy), Parkinsonism, FTDP-17 (Pronto-Temporal Dementia with Parkinsonism linked to chromosome 17), Familiar British Dementia, Prion Disease (Jacob Creutzfeld Disease) and Picks Disease.

The term "tauopathies" as used herein refers to pathologies characterised by aggregated tau into paired helical filaments leading to neurodegeneration.

In an equally preferred embodiment of the invention MARKK are used for the preparation of a pharmaceutical composition for the treatment of pathologies characterised in that the reduced phosphorylation of tau protein results in at least one to two times more tau protein bound to microtubules compared to healthy cells.

The inventors were able to find clear evidence that hypophosphorylation of tau protein is as much lethal to cells as hyperphosphorylation. When tau is overexpressed as in Alzheimer Disease neurons (IQBAL, K. & GRUNDKE-IQBAL, I. 1997) one finds at least one to two times higher tau content compared to healthy neurons. This excess tau protein binds to the MT surface and leads to severe inhibition of the movement of the kinesin dependent transport of vesicle and organelles along the MT in anterograde direction towards the synapse (EBNETH, A. et al. 1998; STAMER, K. et al. 2002; LEWIS, J. et al. 2000). This kind of inhibition results in impaired distribution of vesicles and organelles leading to the symptoms of axonal degeneration and loss of neuronal polarity, similar to that found in cells with hyperphosphorylated tau protein (see Example 9).

It has further been shown, that overexpression of MARK in adult and differentiated mammalian cells (CHO) leads to microtubule breakdown and cell death by hyperphosphorylation of MAPs, but expression of MARK in developing neurons stimulates the development of neurites during differentiation by inducing MT dynamics (DREWES, G et al. 1997; EBNETH, A et al. 1999; BIERNAT, J. et al. 2002) by tau phosphorylated at KXGS sites.

In preferred embodiments, other pathologies exhibiting increased binding of tau to MTs resulting in at least one to two times more tau bound to MTs compared to healthy neurons that can be treated by use of MARKK are selected from the group of Tauopathies consisting of CBD (Cortical Basal Disease), PSP (Progressive Supra Nuclear Palsy), Parkinsonism, FTDP-17 (Fronto-Temporal Dementia with Parkinsonism linked to chromosome 17), Familiar British Dementia, Prion Disease (Jacob Creutzfeld Disease) and Picks Disease.

According to one aspect of the present invention MARKK or MARKK antagonists that have a promoting or inhibiting effect on the phosphorylation of MARK are used for the treatment of said pathologies.

The effect of MARKK or MARKK antagonists on the degree of phosphorylation of tau protein can be determined *in vivo* by methods comprising both established AD cell culture and AD transgenic mice model systems overexpressing tau and tau mutants (VOGELSBERG-RAGAGLIA, M. et al. 2002; LEWIS, J. et al. 2000; GOTZ, J. et al. 2001).

Efficacy of administration in humans can be assayed *in vivo* by determining the amount of tau protein present in cerebrospinal fluid of patients as a sign of neuronal cell death via immunochemical analysis (Elisa, Western blot, immunoprecipitation, VANDERMEEREN, M. et al. 1993), especially using kits designed for the detection of tau concentration in cerebrospinal fluid.

Further, the effect of the administered MARKK or a MARKK antagonist according to the invention on the tauopathy and its symptoms can be monitored non-invasively by performing cognitive and behaviour tests (e.g. MMSE, Minimental state examination; FOLSTEIN, M.F. et al. 1975) and comparing the results with the results obtained before the beginning of the therapy.

In another aspect of the invention the pathology characterised by increased or reduced phosphorylation of tau protein at KXGS sites is cancer.

Increased or decreased phosphorylation of MAPs may be present in all cancer cells where mitosis is increased by the increased dynamics of microtubules. Increased dynamics of microtubules ensues rearrangement of microtubules in the cell thereby enabling cell proliferation. Regulation of MARK and MAPs via phosphorylation therefore has important implications on the generation of cancer. In this context it has previously been shown in several types of carcinoma, that a distinct marker protein normally present in healthy cells disappears when the cancer starts to develop (PARSA, I. 1988). The inventors identified this protein to be MARK3.

Activation of MARKS is achieved by phosphorylation of a single residue within the activation loop by MARKK (T210 MARK1, T208 MARK2, T211 MARK3, T214 MARK4). It requires S212 to be present, but not phosphorylated. A fraction of MARK in brain tissue is doubly phosphorylated (at T208/S212), reminiscent of the activation of MAP kinase; however, the phosphorylation of the second site in MARK (S212) is inhibitory (see Example 5).

MARKK, isolated as the active complex from brain has a mass of ∼330 kD which would be compatible with a complex of 2-3 MARKK molecules, or with a complex between MARKK, scaffolding proteins, and other cofactors. MARKK contains predicted amphipathic helices in the spacer and tail domains which would favour protein-protein interactions.

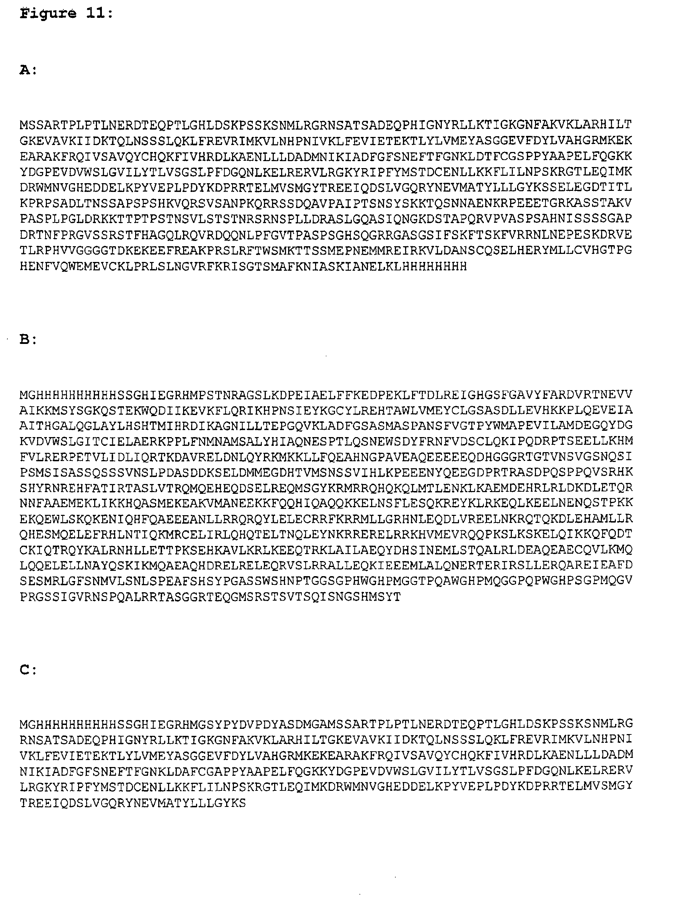

In the present application the term "MARKK" is used to refer to a kinase comprising the amino acid sequence provided in SEQ ID NO: 3 (Figure 11B) or a homologue, derivative or variant thereof that is at least 70%, preferrably at least 80% and most preferred at least 90% identical to the amino acid sequence provided in SEQ ID NO: 3. Further fragments are included which have the ability to phosphorylate MARK. According to a preferred embodiment of the invention sequence homology is determined using the BLAST software.

Homologue polypeptides are such substances that differ from the amino acid sequence provided in SEQ ID NO: 3 by exchange of one or more amino acids or by deletion or insertion of on one or more amino acids resulting in sequence homology of at least 70%, preferred at least 80% and most preferred at least 90%. Derivatives according to the invention are such polypeptides, wherein one or more amino acids of the amino acid sequence provided in SEQ ID NO: 3 are replaced by a stereo isomer (i.e. replacement of one or more L amino acids by D amino acids) or a corresponding modified amino acid. Modified amino acids are such amino acids, wherein side chains are chemically modified compared to naturally occurring amino acids, for example by glycosylation, phosphorylation, methylation etc. Such modifications are well known in the art.

In accordance with the present invention a compound is referred to as a MARKK antagonist, if it inhibits the kinase activity of MARKK in vitro as determined by using the activity assay as described in TIMM, T. et al. 2003.

In a preferred embodiment the MARKK antagonist is selected from the group consisting of an antibody, an antisense construct, a siRNA, a peptide that specifically binds to MARKK, an agent or an enzyme, that is capable of substantially inhibiting the phosphorylating activity of MARKK, and a phosphatase.

In a further preferred embodiment the inhibitor of MARKK is the protein kinase PAK5 (NCBI accession number AB040812, GI:7649809) which the inventors isolated from a fetal brain cDNA library as an interaction partner of MARK by using a yeast two-hybrid approach with MARK2 as bait. Full-length PAK5 was subsequently cloned by PCR from this fetal cDNA library. Upon transfection into CHO cells PAK5 stabilised the microtubule network and destabilised the actin network (Fig. 10 a & b). When MARKK was co-transfected with PAK5 in CHO cells, both kinases co-localised in the cytosol, and the destructive effect of MARKK on microtubules was relieved (Fig. 10 c). This inhibition of MARKK by PAK5 was also shown *in vitro* in a kinase assay where recombinant kinases MARKK and PAK5 were purified from Sf9 cells and tested with regard to their ability to phosphorylate MARK (Fig. 10 d).

In an equally preferred embodiment of the invention the MARKK antagonist is an antibody.

In one aspect of the invention MARK or MARKK antagonists are used for the preparation of a pharmaceutical composition that is administered via any suitable administration route (e.g. see TAMURA, K. et al. 2003).

In an preferred embodiment of the invention the pharmaceutical composition is administered orally or parenterally.

As has been pointed out above, the deregulation of the degree of the phosphorylation of tau protein can lead to the development of a variety of pathologies named tauopathies in the scientific community. Accordingly, a pharmaceutically effective amount of MARKK or a MARKK antagonist will restore the balance between tau bound to MTs and unbound tau as present in healthy cells thereby reversing the symptoms of the respective tauopathy. The amount of MARKK or the MARKK antagonist and the scheme of administration will be determined by the physician handling the respective case.

In a further preferred embodiment of the invention MARK or MARKK antagonist are used for the preparation of a pharmaceutical composition that is administered as part of a sustained release formulation resulting in slow release of the compound following administration. Such formulations are well known in the art and may generally be prepared using well known technology and administered, for example, by implantation at the desired target site, e.g. in the brain (SHELEG, S.V. et al. 2002).

In another embodiment of the invention the enzyme, peptide, agent or antibody capable of substantially inhibiting the phosphorylating activity of MARKK is a MARKK antagonists which is administered using genetically manipulated viruses selected from the group consisting of Adenoviruses and Lentiviruses deficient in reproduction coding for the respective MARKK antagonist. The use of a variety of viruses for treating neurodegenerative diseases is well known in the art (BAEXELANDT, V. et al. 2000; KASPAR, B.K. et al. 2003).

The invention further relates to pharmaceutical compositions for the treatment of pathologies characterised by increased or reduced phosphorylation of MARK and/or tau proteins.

In a preferred embodiment of the present invention, the pharmaceutical composition for the treatment of pathologies characterised by increased or reduced phosphorylation of MARK and/or tau proteins comprises MARKK antagonists or MARKK. In accordance with the present invention a compound is considered to be a MARKK antagonist, if the compound inhibits the kinase activity of MARKK leading to deactivation of MARK. The MARKK antagonist can for example be an antibody, an antisense construct, a siRNA, a peptide that specifically binds to MARKK, an agent or an enzyme, that is capable of substantially inhibiting the phosphorylating activity of MARKK, and a phosphatase.

In accordance with the present invention the pharmaceutical composition comprises MARKK or MARKK antagonists for the treatment of pathologies characterised by increased or reduced phosphorylation of MARK and/or tau proteins and further a pharmaceutically acceptable carrier and/or diluent.

The following Examples illustrate the effectivity of MARKK and MARKK antagonists in the treatment of pathologies characterised by increased or reduced phosphorylation of MARK and/or tau proteins.

### Labelling of proteins with FSBA

Proteins were transferred into PBS using a FastDesalting™ column (Pharmacia) and incubated overnight with FSBA (5'-p-fluorosulfonylbenzoyladenosine, ATP-Binding Protein Detection Kit, Boehringer Mannheim) at a final concentration of 1 mM. The reaction was stopped with 5 mM -mercaptoethanol at RT for 1 hour and dialysed against 50 mM Tris-HCl pH 7.5, 200 mM NaCl, 1 mM benzamidine, 1 mM -mercaptoethanol, 1 mM PMSF, 50 % glycerol.

### Antibodies

Rabbit antisera were raised against the N-terminal peptide of MARK, the lip peptide of MARK phosphorylated at T208 or phosphorylated at S212 (referred to as pT208-lip antibody and pS212- lip antibody). Secondary antibody for blotting was HRP-conjugated goat anti-rabbit (Dianova). Antibody 12E8 against phosphorylated S262 of tau was a gift of P. Seubert (Elan Pharma). Rat monoclonal antibody YL1/2 against tubulin was from Serotec, antibody against MARKK/TAO-1 was from BD Biosciences (Palo Alto, CA), anti- -actin was from Sigma, and rabbit polyclonal antibody against FSBA was from Boehringer Mannheim.

### Brief description of the Figures

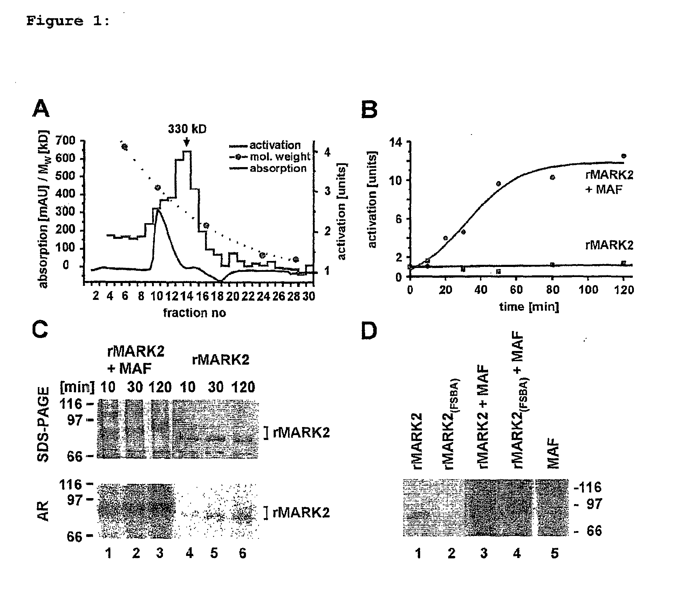

**Figure 1:** Activation of MARK2.
   A: Protein and activity profile of G200 gel filtration column. Fractions of brain extract were tested for the activation of MARK to phosphorylate the TR1 peptide (NVKSKIGSTENLK, SEQ ID NO: 1). Most of the protein elutes around fraction 10 (bottom), but the activity peaks in fractions 13-14 (top, arrow). Calibration of column is shown as dotted line (catalase, ferritin, aldolase, serum albumin, ovalbumin).
   B: Activation of MARK2 by MARK-activating factor. Bottom: Recombinant MARK2 (sequence depicted in Figure 11a, SEQ ID NO: 2) alone phosphorylates the TR1 peptide at a constant low rate (55 nmol/min/ mg). With MAF (top) the activity of MARK2 increases ∼10-fold.
   C: Phosphorylation of MARK2 increases with activation by MAF. Top left, SDS gel showing the Mr shift (from 88 to 97 kD) during activation by MAF. Top right, MARK2 alone shows no Mr shift, despite some autophosphorylation. Bottom left: Autoradiogram of gel showing the increase in phosphate bound to MARK2, mostly at T208 (see below). Bottom right: MARK2 alone shows little autophosphorylation (outside the catalytic domain, data not shown).
   D: Lanes 1 & 2: autoradiograms of MARK2 in active and inactive states. Activity was blocked by covalent modification of ATP binding site by FSBA; Lanes 3 & 4: incorporation of phosphate into active and inactive MARK2 by MAF, illustrating that MAF contains a kinase independently of the activity of MARK2; Lane 5: control, MAF alone shows no phosphate incorporation at the position of MARK2.

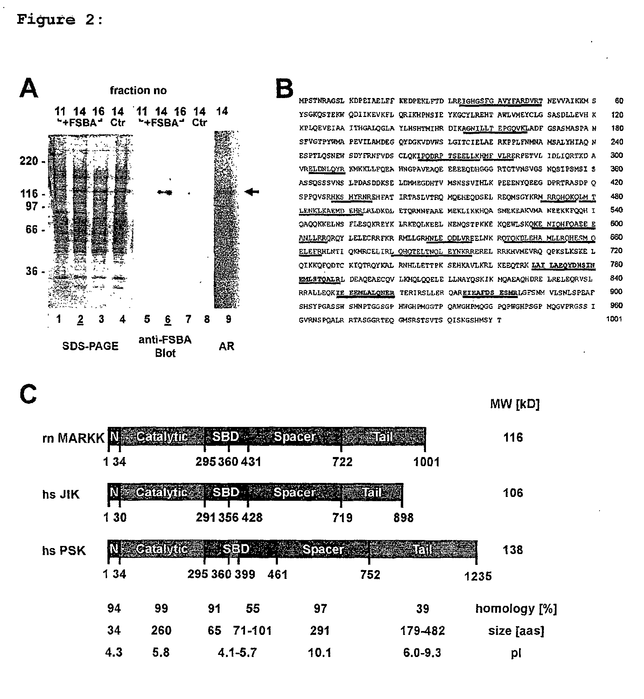

**Figure 2:** Sequence and domain structure of MARKK.
   A: Identification of MARKK.
      Lanes 1-4 show SDS gel of fractions 11, 14, 16 (with FSBA), and 14 (control without FSBA) from G200 column (fraction 14 contains the highest activity, see Fig. 1A). Lanes 5-8 are blots with an antibody against FSBA. Only one band at 134 kD is labelled in the active fraction (lane 6, fraction 14), the control without FSBA shows no signal (lane 8). For comparison, lane 9 shows an autoradiogram of an active fraction (same sample as in lane 4) with a band of active MARKK at Mₐₚₚ= 134 kD, labelled because of autophosphorylation.
   B: Sequence of MARKK from a rat brain cDNA library.
      Peptides identified by sequencing and mass spectrometry are underlined. The sequence is equivalent to that of TAO-1 (accession code AF084205) or the human sequence KIAA1361 (AB037782). The 4 bold peptides are unique for TAO-1 and distinguish the kinase from PSK/TAO-2 and JIK.
   C: Domain structure of MARKK/TAO-1 and comparison with other kinases of high homology (JIK and PSK) in the Ste20 family (N = N-terminal header domain, C = catalytic domain, SBD = substrate binding domain, S = spacer domain, T = tail domain).
      Extended coiled-coil sequences are predicted in the spacer and tail domains (S430-A630, K730-F900). The substrate binding domain of PSK/TAO-2 binds to the N-terminal header domain of its substrate MKK3, the kinase upstream of p38 MAP kinase. However, it is unclear whether an analogous interaction occurs with MARKK and MARK since the corresponding domains are poorly conserved.

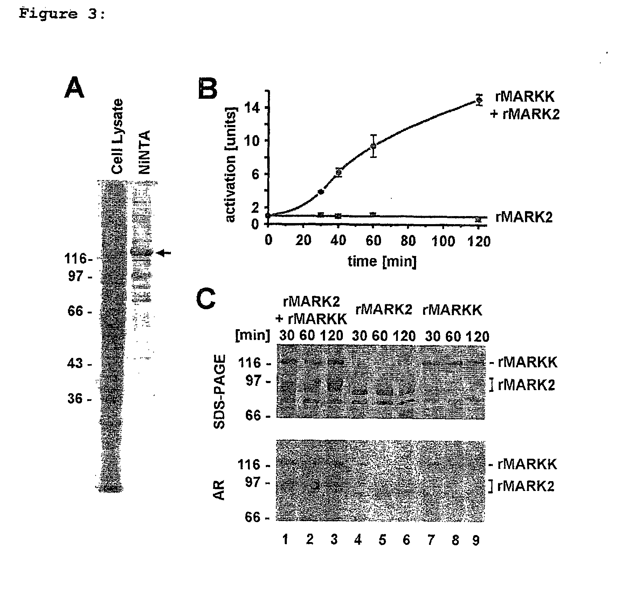

**Figure 3:** Activity of recombinant MARKK.
   A: Expression of recombinant MARKK (His10-tagged at the N-terminus, Fig. 11 b, SEQ ID NO: 3) in Sf9 cells.
      Lane 1: SDS gel of Sf9 cell lysate; lane 2: protein retained on Ni-NTA column, showing enrichment of MARKK at Mₐₚₚ = 134 kD (slightly larger than the predicted 119 kD).
   B: Activation of MARK2 by recombinant MARKK, measured by the phosphorylation of the TR1 peptide (SEQ ID NO: 1) by MARK2. Bottom curve (squares): Recombinant MARK2 (SEQ ID NO: 2) alone phosphorylates the peptide at a constant low rate. MARKK activates MARK2 more than 16-fold after 2 h (top curve, circles).
   C: Phosphorylation of MARK2 increases with activation by MARKK. Top left (lanes 1-3): SDS gel showing the gradual shift of MARK2 in Mr (from 88 to 97 kD) during activation by MAF; Top middle (lanes 4-6): MARK2 alone shows no shift in Mr, despite some autophosphorylation; Top right (lanes 7-9): MARKK alone shows no shift in Mr either; Bottom left: Autoradiogram of gel showing the increase in phosphate bound to MARK2, mostly at T208 (see below); Bottom middle: MARK2 alone shows little autophosphorylation (at sites outside the catalytic domain, data not shown); Bottom right, MARKK shows some autophosphorylation (note that the lower band co-migrating with MARK2 is a coincidence and probably due to degradation of MARKK).

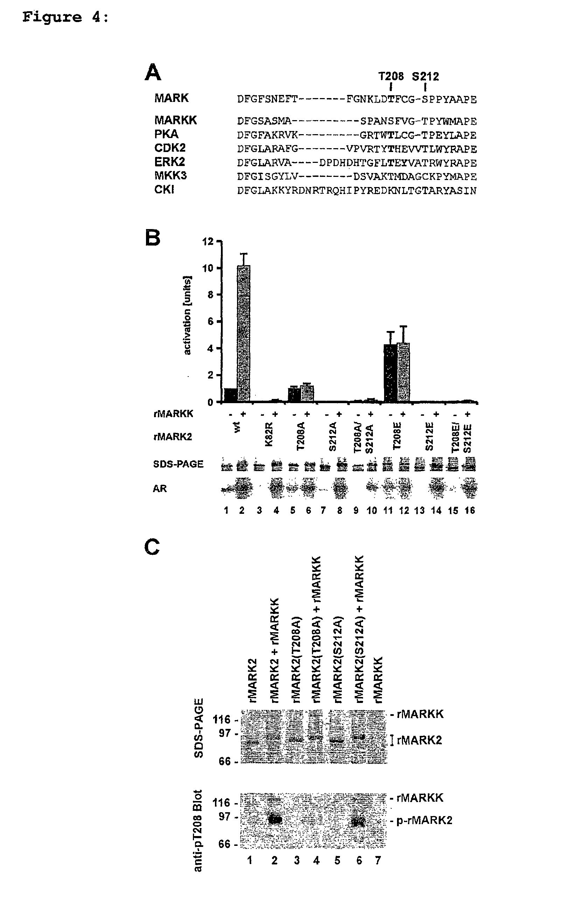

**Figure 4:** Regulation of MARK.
   A: Activation loop of MARK2 and other kinases.
      Known phosphorylation sites are in bold.
   B: Influence of activation loop on MARK2 activity.
      Top: Bar 1: MARK2 alone has a low basal activity of 55 nmol/min/mg; Bar 2: activation by MARKK is 10-fold; Bars 3 & 4: the K82R mutant has no activity and cannot be activated; Bars 5, 6: the T208A mutant has the same activity as wild type, irrespective of MARKK, indicating that the phosphorylation of T208 is important for activation; Bars 7-10: The S212A mutant and the T208A/S212A mutant of MARK2 show no activity at all, indicating that S212 is important for basal activity, independently of whether T208 is phosphorylated by MARKK or not; Bars 11 & 12: the T208E mutant has a fourfold higher activity than the wild type but cannot be further activated by MARKK; Bars 13-16: the S212E mutant and the T208E/S212E mutant of MARK2 show no activity. Middle: SDS gel showing the shift in Mr of all MARK2 mutants after phosphorylation with MARKK as well as the wild type MARK2. The T208E mutant has a lower electrophoretic mobility even without phosphorylation (lane 11). Bottom: Autoradiograms showing that autophosphorylation corresponds to activity (odd lanes). All proteins become phosphorylated upon incubation with MARKK (even lanes). This indicates at least one phosphorylation site outside the regulatory loop lacking influence on activation.
   C: Phosphorylation of MARK2 and mutants (T208A or S212A) during activation by MARKK.
      Top: SDS gel, bottom: blot with antibody against phospho-Thr. Odd lanes without MARKK, even lanes with activation by MARKK. Lane 1: MARK2 alone; Lane 2: MARKK phosphorylates MARK2 with a shift in Mr (top), and with a prominent phospho-Thr site recognised by the pT208 antibody; Lane 3: T208A mutant of MARK2; Lane 4: MARKK causes an Mr shift of MARK2-T208A (top), but there is no phospho-Thr reaction, concomitant with the loss of activation (see B. above). It also shows that T208 is not responsible for the Mr shift; Lane 5: S212A mutant of MARK2; Lane 6: Phosphorylation by MARKK of MARK2-S212A creates the phospho-Thr epitope at T208 (but without activity). There is an Mr shift that occurs independently of S212; Lane 7: Control, MARKK alone shows no signal with the antibody against phospho-T208.

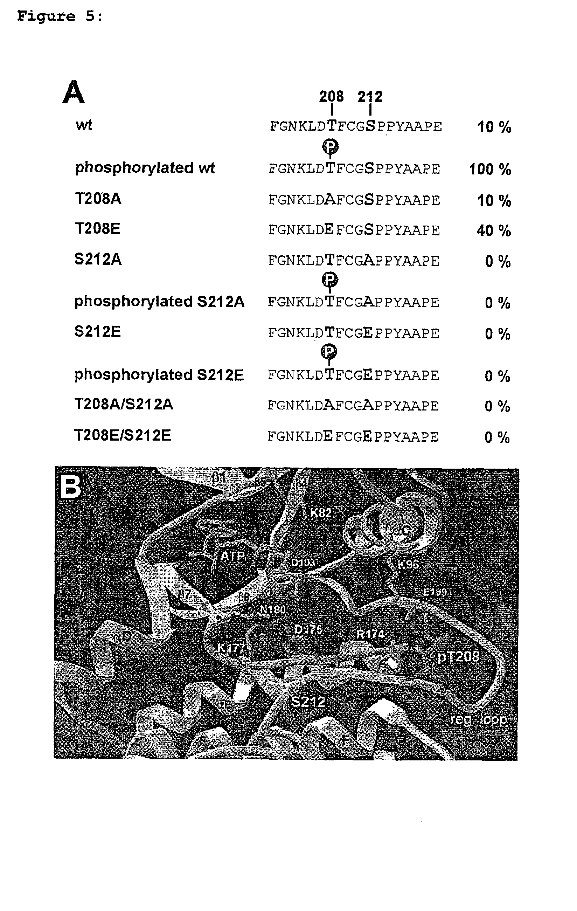

**Figure 5:** Regulation of MARK.
   A: Sequence F202-E219 around the activation loop of MARK2, with T208 and S212 highlighted.
      The wild-type sequence allows only basal activity (10%) which rises 10-fold upon phosphorylation of T208 by MARKK. Mutant T208A allows only basal activity, T208E shows partial activation. Phosphorylation of S212 is not required for activation, but any mutation of S212 (A or E) destroys activity, showing that S212 must be present for activity while mutants of S212 are inactive even when T208 is phosphorylated.
   B: Structural model of the regulatory loop of MARK2 using PKA as a template (SWISSMODEL, http://www.expasy.ch/swissmod/SWISS-MODEL.html).
      Catalytic (violet) and activation loops (light green) lie between the small lobe (mainly -sheets, light yellow) and the large lobe (mainly -helices, pink). Phospho-T208 (red) contacts K96, R174 (light blue) and E199 (orange) via hydrogen-bonds stabilising the regulatory loop. S212 (yellow) forms a hydrogen bond with K177 (light blue) in the catalytic loop which also stabilises the -phosphate of ATP (green) during the phospho-transfer and is conserved in S/T-kinases. The catalytic D175 (red) is stabilised by N180 (light blue). K82 (light blue) fixes the - and - phosphates of ATP, together with the Mg⁺⁺-binding D193 (orange). Note that in PKA and MAP kinase the phosphorylation corresponding to T208 stabilises the activation loop and opens the substrate pocket (by interacting with the residues corresponding to K96, R174 and E199 in MARK, JOHNSON, L.N. et al. 1996), whereas the conserved DFG motif (193-195) and S212 form fixed points for anchoring this flexible loop (contributing a hydrogen bond with K177). In this scheme, the phosphorylation of S212 would be inhibitory because it would disrupt the fixation of the loop. It is also possible that the Glu in this position interferes with the binding of the substrate to the catalytic cleft (SCOTT, J.W. et al. 2002). Mutation of the equivalent to S212 in JIK results in a dramatic decrease of activity (TASSI, E. et al. 1999). The doubly phosphorylated peptide in brain-derived MARK2 probably come from an inactive fraction. The kinase responsible for phospho-S212 is unknown, but the inventors note that none of the kinases applied in combination with MARKK was able to phosphorylate S212 in MARK2, and there was no change in activity.

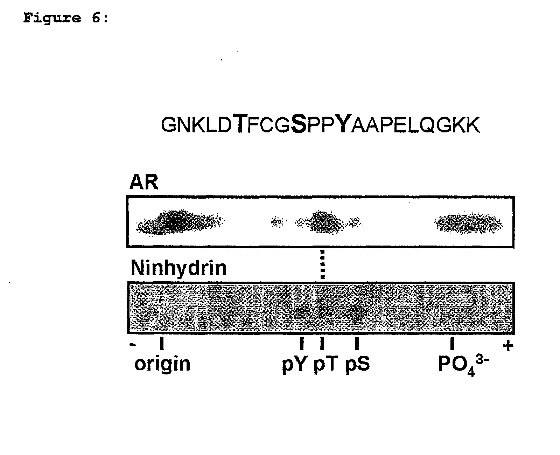

**Figure 6:** Phosphorylation of MARK.
   Phosphorylation of peptide ₂₀₃GNKLDTFCGSPPYAAPELQGKK₂₂₄ (SEQ ID NO: 4, from the activation loop of MARK2) by MARKK. Top: Phospho-amino acids generated by hydrolysis with HCl, separated by thin-layer-chromatography, and imaged by autoradiography. Bottom: standards of phosphorylated tyrosine, threonine, and serine stained with ninhydrin. The MARK2-peptide is almost exclusively phosphorylated at T208.
**Figure 7:** MARKK-MARK2 interactions in Sf9 cells.
   A: Left: Expression of MARK2 without or with MARKK (lanes 1 & 2) in Sf9 cells after baculovirus transfection.
      Equal amounts of Sf9 cell lysates were probed by Western blotting with antibody against HA-tag (recognises MARK2) and antibody TAO1 (recognises MARKK).
      Right: Immunoprecipitation of MARK2 from Sf9 cells expressed alone (lane 1) or with MARKK (lane 2).
      Equal amounts of cell lysate were immunoprecipitated with beads coupled to HA-tag antibody and equal bead samples were loaded on SDS PAGE. Note that the amount of MARK2 is similar in both samples.
   B: Stimulation of MARK2 activity by MARKK in Sf9 cells.
      MARK2 was immunoprecipitated from Sf9 cells transfected with BV-MARK2 (white bars) or a mixture of BV-MARK2 and BV-MARKK (black bars) and subjected to kinase assay with tau substrate (TR1 peptide). The incubations with 0.5 mM ATP were done for 1.4 or 24 h. Note that MARKK enhances MARK2 activity 3-fold at 24 h.
   C: Co-immunoprecipitation of MARKK with MARK2 or dominant negative MARK2 (T208A/S212A) from transfected Sf9 cells: MARK2 (lane 1), dnMARK2 (lane 2), MARKK (lane 3), MARK2/MARKK (lane 4), dnMARK2/MARKK (lane 5).
      Immunoprecipitation was done with HA-tag antibody against HA-tagged MARK2 and analysed by Western blots using either HA-tag antibody (against MARK2) or TAO1 antibody (against MARKK). Note that MARKK co-immunoprecipitates only with dominant negative mutant of MARK2 (lane 5). The expression of MARKK in total extracts was at comparable levels (top, lanes 3-5).

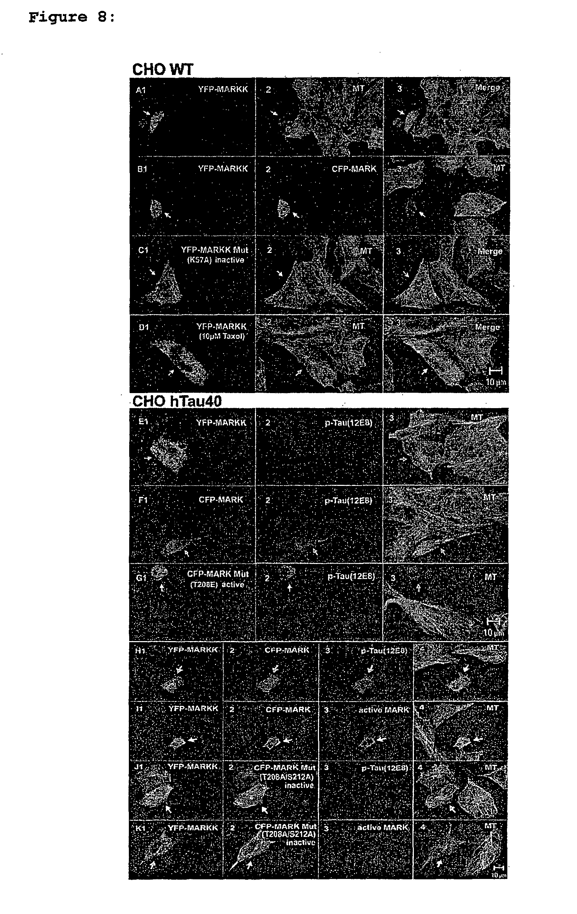

**Figure 8:** Active MARKK in cells destroys microtubules via activation of MARK and phosphorylation of Tau.
   A: CHO cell transfected with YFP-MARKK, fixed and observed by confocal microscopy (A1, arrow), staining of microtubules with antibody YL1/2 and Cy5-secondary antibody (A2), merged images (A3).
      Note that the transfected cell (arrow) lost its microtubule network, rounded up and appears smaller.
   B: Cell co-transfected with YFP-MARKK (B1, arrow) and CFP-MARK (B2) or staining of microtubules with YL1/2 (B3).
      Note the shape change and destruction of microtubules in the co-transfected cell, similar to A.
   C: CHO cell transfected with inactive YFP-MARKKK^{57A} imaged as above (C1, YFP fluorescence, C2 microtubules, C3 merged).
      The transfected cell (left, arrow) retains its microtubule network.
   D: Cell transfected with YFP-MARKK (Dl, arrow), and microtubules stabilised by 10 M taxol (D2), merged images (D3).
      Note that taxol prevents the destabilisation of microtubules by MARKK.
   E: Cell stably transfected with tau and transiently transfected with YFP-MARKK.
      Staining of YFP-MARKK (E1, arrow), phospho-tau (KXGS motifs) by antibody 12E8 (TRITC) (E2), microtubules (E3). Note that elevated tau stabilises microtubules against the effect of MARKK.
   F: Cell stably transfected with tau and transiently transfected with CFP-MARK.
      Staining of CFP-MARK (F1, arrow), phospho-tau (KXGS motifs) (F2), microtubules (F3). Note that elevated tau stabilises microtubules against the effect of MARK.
   G: Cell stably transfected with tau and transiently transfected with CFP-MARK^{T208E}.
      Constitutively active MARK (G1, arrow) phosphorylates tau at KXGS motifs (G2), and destroys microtubules (G3).
   H: Cell stably transfected with tau and transiently co-transfected with YFP-MARKK and CFP-MARK.
      Staining of YFP-MARKK (H1, arrow), CFP-MARK (H2), phospho-tau (KXGS motifs) (H3), and microtubules (H4). Note that the combined effect of MARKK and MARK destabilises microtubules in spite of tau.
   I: Experiment similar to H but highlighting active MARK by the antibody SA6941-TRITC against the phosphorylated activation loop (I3, arrow).
      YFP-MARKK (I1), CFP-MARK (I2), active MARK (TRITC) (I3), and microtubules (Cy5) (I4).
   J: Cell stably transfected with tau and transiently co-transfected with YFP-MARKK and CFP-MARK-mutant (T208A, S212A).
      Staining as in H. Microtubules remain intact because the MARK mutant cannot be activated.
   K: Experiment similar to J, i.e. co-transfection with YFP-MARKK (K1, arrow), CFP-MARK mutant (K2), and staining with antibody against active MARK (K3).
**Figure 9:**
   A: PC12 cells differentiated with NGF (72 h, 100 ng/ml) and immunostained for MARKK (A1, antibody TAO-1), MARK (A2, antibody SA2118), and merged image (A3).
   Note that endogenous MARKK and MARK co-localize, notably beneath the plasma membrane and at growth cones (arrows).
   B: PC12 cells differentiated as above, immunostained for phospho-KXGS-tau (B1, antibody 12E8), MARK (B2), and merge (B3).
   Note that endogenous phospho-KXGS-tau co-localises with MARK, especially near the plasma membrane and the growth cones.
   C: PC12 cells differentiated as above and immunostained for actin (C1, oregon-green-phalloidin), MARK (C2), and merge (C3).
   Note that endogenous MARK co-localises with actin at growth cones.
   D: PC12 cells differentiated with NGF (48 h, 100 ng/ml). D1, mock transfected cell, stained for endogenous MARKK. D2, phase image.
   E: PC12 cells transfected with RNAi against MARKK exposed to NGF as above.
      Note that MARKK is largely suppressed (E1), and the cells cannot differentiate (phase image, E2).
   F: Western blot of PC12 cells after NGF-differentiation, mock-transfected (left) or with RNAi against MARKK (right). Note that expression of MARKK is suppressed.
   G: Quantification of effects of RNAi against MARKK (see D, E). The fraction of cells with neurites decreases from ∼80% to 20% upon treatment with RNAi.
**Figure 10:**
   A: CHO cells transfected with YFP-PAK5 were fixed, stained for actin (labelled by rhodamine-phalloidin), and observed by confocal microscopy.
      Note that the presence of active PAK5 in cells leads to the dissolution of actin stress fibers (see A2, arrow).
   B: CHO cells transfected with YFP-PAK5 were stained for microtubules (labelled by antibody YL1/2 and TRITC-secondary antibody).
      Note that the presence of active PAK5 in cells leads to the stabilisation of the microtubule network (see B1, arrow).
   C: CHO cells co-transfected with YFP-MARKK and PAK5-myc were fixed, stained for PAK5 (myc-tag antibody with Cy5-secondary antibody) and co-stained for microtubules (labeled by antibody YL1/2 and TRITC-secondary antibody).
      Note that PAK5 inhibits MARKK and thus stabilises the microtubule network (see C4, arrow). This is in contrast to Fig. 8a where MARKK, transfected alone into CHO cells, destroyed the microtubule network and led to the rounding-up and death of the cell.
**Figure 11:**
   Sequences
   A: Amino acid sequence of recombinant MARK2 (SEQ ID NO: 2)
   B: Amino acid sequence of recombinant MARKK (SEQ ID NO: 3)
   C: Amino acid sequence of recombinant MARK-NA2 (SEQ ID NO: 5)

### EXAMPLE 1

### Purification of MARK-activating factor (MAF)

Porcine brains (1 kg) were homogenised in 3 L of buffer A (50 mM Tris-HCl pH 8.5, 150 mM NaCl, 50 mM NaF, 5 mM MgCl₂, 3 mM EGTA, 1 mM PMSF, 2 mM benzamidine, 1 mM Na₃VO₄, 1 mM DTT, 1 mM NaATP, 4 mM CHAPS, 0.1% NP40, 10 M pepstatin A) and spun at 12,000 g for 1.5 h. The supernatant was loaded onto a Whatman P11 column in buffer B (25 mM MES pH 6.5, 100 mM NaCl, 2 mM EGTA, 50 mM NaF, 1 mM PMSF, 1 mM benzamidine, 1 mM Na₃VO₄, 1 mM DTT, 0.1% NP40, 5% glycerol, 10 M pepstatin A), washed and eluted with 300 mL of buffer B with 750 mM NaCl in total. The eluate was dialysed against buffer C (50 mM Tris-HCl pH 8.2, 100 mM NaCl, 5 mM EGTA, 10 mM NaF, 1 mM PMSF, 1 mM benzamidine, 1 mM DTT, 0.1% NP40, 5% glycerol), loaded onto Q-sepharose, and eluted with a gradient from 0.1 to 0.6 M NaCl.

Fractions were assayed by phosphorylation of the synthetic tau peptide TR1 (SEQ ID NO: 1). The active pool (40 mL) was desalted on Sephadex G25 equilibrated with buffer D (25 mM MES pH 6.5, 100 mM NaCl, 2 mM EGTA, 10 mM NaF, 0.5 mM PMSF, 1 mM benzamidine, 1 mM Na₃VO₄ ,1 mM DTT, 5 % glycerol, 0.1% NP40) and loaded onto a Mono S HR10/10 column. After elution with a gradient from 0.1 to 0.6 M NaCl active fractions (10 ml) were desalted on Sephadex G25 equilibrated with buffer E (40 mM HEPES pH 7.3, 100 mM NaCl, 2 mM MgCl₂, 1 mM NaATP, 0.5 mM PMSF, 1 mM benzamidine, 1 mM -mercaptoethanol, 5% glycerol, 0.1% NP40) and loaded onto a NiNTA-column saturated with 7.5 mg of recombinant MARK2-NA2 (containing residues 1-368, an N-terminal His12-tag, T208 and S212 in the regulatory loop mutated to alanine, SEQ ID NO: 5, Fig. 11 c) and eluted with a step from 0.1 to 1.0 M NaCl. The active pool (3 mL) was concentrated twofold with a molecular filter (Amicon exclusion size 10 kD) and the sample was gel filtered (Pharmacia Superdex G200; 16 x 600 mm) in buffer D. Active fractions were dialysed against buffer F (50 mM Tris-HCl pH 8.2, 200 mM NaCl, 1 mM PMSF, 1 mM benzamidine, 1 mM DTT, 0.1% NP40, 50% glycerol) and stored at -20°C. Pellets were dissolved in sample buffer and electrophoresed on an 8 % SDS-polyacrylamide gel. After staining with Coomassie G250 the putative kinase band was excised and analysed by peptide sequencing and mass spectrometry as described (GEVAERT, K. et al. 2001).

### EXAMPLE 2

### Kinase assay

Kinase activities were assayed in 50 mM Tris-HCl pH 7.5, 5 mM MgCl₂, 2 mM EGTA, 0.5 mM PMSF, 0.5 mM DTT, 0.5 mM benzamidine for 30 minutes at 30°C. Final concentration of [³²P] ATP (3.7 × 10⁷ MBq/mol) and substrate peptide were 100 M. As substrate a peptide derived from the first repeat of tau protein containing S262 in the KXGS motif (TR1-peptide NVKSKIGSTENLK, DREWES, G. et al. 1997, SEQ ID NO: 1) was used. Reactions were stopped by addition of half the volume of 30 % (w/v) TCA. After centrifugation the supernatant was applied to phosphocellulose-paperdiscs, washed with phosphoric acid (0.1 M), dried by air and radioactivity was measured in a scintillation counter (Tricarb 1900 CA, Packard). Data show averages of 3 experiments (bars = s.e.m.).

Extracts from pig brain were fractionated and tested for their activation of MARK2. The highest gain (-80-fold) was achieved by affinity chromatography using the catalytic domain of MARK2 (residues 1-368, for MARK2 see SEQ ID NO: 2), inactivated by mutations T208A and S212A (Data not shown). Fig. 1A compares the elution profile from the G200 gel filtration column (bottom) with MARK activation (top). The peak (fractions 13-14) corresponds to a ~330 kD mass, several times larger than the activating kinase (see below), suggesting that the MARK activating factor (MAF) is a complex of proteins. Next, the activation of MARK2 by fraction 14 was tested. MARK2 alone had a low basal activity (Fig. 1B, bottom), but with MAF the activity increased ∼10 fold (upper curve). This was accompanied by the phosphorylation of MARK2, as expected for the activation mechanism. The SDS gel of Fig. 1C (top) shows that the Mr of MARK2 shifts upward from ∼88 to ~97 kD in the presence of the activating factor (left), but not in its absence (right); this shift is also found with natively active MARK2 (DREWES, G. et al. 1997). Likewise, the incorporation of phosphate is also strongly increased by the activating factor (Fig. 1C, bottom).

To prove that the activating factor in the mixture of fraction 14 (Fig. 1A) indeed contains a kinase, the fractions were tested for activity after incubation of MARK2 with FSBA (Fig. 1D) which binds to ATP sites of proteins and blocks kinase activity (ANOSTARIO, M. et al. 1990). The autoradiogram of Fig. 1D shows that untreated MARK2 becomes autophosphorylated (lane 1, note that this affects sites not crucial for activation, e.g. T58, S275, T276, outside the catalytic site, as judged by a peptide array, data not shown). There is no autophosphorylation when MARK2 is incubated with FSBA prior to the kinase assay, and no incorporation of radioactivity (lane 2), indicating that the kinase is no longer active.

When MARK2 not treated with FSBA is exposed to MAF there is strong incorporation of phosphate into MARK2 (Fig. 1D, lane 3), including the activation loop (see below). The activating factor can phosphorylate MARK2 even when MARK2 is inactive due to FSBA binding (lane 4). This rules out the possibility that MAF is a cofactor enhancing the autophosphorylation of MARK2 and argues that MAF indeed contains a kinase phosphorylating MARK2 which is therefore termed MARKK (MARK kinase).

To clarify which protein in the MARK2-activating fraction was the kinase, fractions 11-16 of the G200 eluate were labelled with FSBA. The proteins were separated on a SDS gel, blotted and probed with an antibody against FSBA (Fig. 2A). This revealed a single band at Mr -134 kD, occurring only in the highly active fraction 14 (lane 6), and only when FSBA was present (compare lane 8). This fraction also showed some autophosphorylation (Fig. 2A, lane 9).

The 134 kD band was isolated by preparative SDS gel electrophoresis, digested with trypsin, and 16 peptides were identified by mass spectrometry and sequencing as described (GEVAERT, K. et al. 2001, Fig. 2B).

### EXAMPLE 3

### cDNA cloning

The sequences obtained from the peptide analysis (see example 2) were used for data base screening (EMBL BioInformatics, http://www.ebi.ac.uk/Databases/). Sixteen of the tryptic peptides were found to be part of a kinase named TAO1 (Thousand And One Aminoacids, Genbank AF084205; HUTCHISON, M. et al. 1998) of rat brain origin. Using oligonucleotides which introduce a NdeI-restriction site at the start-codon (SEQ ID NO: 6, 5'-TGA CTG CTC AGC CAT ATG CCA TCA ACT AAC-3') and a BamHI-restriction site after the stop-codon (SEQ ID NO: 7, 5'-CAG CTC CAG CGG ATC CGC CAC TTT CAA TTA TTA-3') the cDNA coding for this kinase was amplified with PCR out of a -ZAP-library (Stratagene). The PCR product of 3050 base pairs was subcloned into the pCR2.1-Vector (TOPO TA Cloning Kit, Invitrogen) for sequencing. For recombinant expression the coding region of the kinase was subcloned into a modified pVL1392-Vector (Pharmingen). A linker containing a N-terminal His10-Tag and an in-frame NdeI-restriction site was inserted at the XbaI-site (SEQ ID NO: 8, 5'-TCT AGA AAT AAT TTT GTT TAA CTT TAA GAA GGA GAT ATA CCA TGG GCC ATC ATC ATC ATC ATC ATC ATC ATC ATC ACA GCA GCG GCC ATA TCG AAG GTC GTC ATA TG-3'), the original NdeI-site was deleted by mutagenesis. Site-directed mutagenesis was performed by QuickChange kit (Stratagene).

The kinase was cloned from a rat brain cDNA bank using primers derived from the 5' and 3' end of the TAO-1 sequence (Fig. 2B & C, SEQ ID NO: 6 & 7). The arrangement of domains is similar to that of MARK, with an N-terminal kinase catalytic domain preceded by a header and followed by spacer and tail domains. Secondary structure predictions reveal extended coiled-coil motifs in the spacer and tail domains (S430-A630, K730-F900).

### EXAMPLE 4

### MARK and MARKK preparation from E. coli and Sf9 cells

Recombinant MARK2 (SEQ ID NO: 2, Fig. 11 a) was expressed in E. coli (DE3 pLys), recombinant MARKK (SEQ ID NO: 3, Fig. 11 b) was expressed in Sf9 cells using the baculovirus system BaculoGold™ (Pharmingen). E.coli or Sf9 cells were lysed in buffer A (50 mM TRIS-HCl pH 8.5, 100 mM NaCl, 50 mM imidazole, 5 mM CHAPS, 1 mM benzamidine, 1 mM -mercaptoethanol, 1 mM PMSF) with a French Press. The supernatant was loaded onto a NiNTA™-Column (Qiagen). After washing with buffer A the protein was eluted with a short gradient of 50-500 mM imidazole. The eluted protein was dialysed against buffer C (50 mM Tris-HCl pH 7.5, 200 mM NaCl, 1 mM benzamidine, 1 mM DTT, 1 mM PMSF, 50% glycerol) and stored at -20°C.

Expression of MARKK in E. coli yielded only degraded and insoluble material, but expression in Sf9 cells resulted in functional protein with an Mr value of 134 kD, as expected (Fig. 3A). The reason for this phenomenon is that unlike E. coli the Sf9 cells contain the machinery for posttranslational modifications, including kinases for keeping MARKK active.

The activation of MARK2 by recombinant MARKK was demonstrated by the same procedures as those used before for MARK-activating factor (Fig. 3B, see Fig. 2B and example 1). MARK2 alone showed a low basal activity of 55 nmol/min/mg, rising to 233 nmol/min/mg at TR1 peptide (SEQ ID NO: 1) concentrations >1.5 mM. Treatment of MARK2 with MARKK lead to a 10 - 15-fold activation (∼800 nmol/min/mg, circles), whereas MARKK alone showed no activity towards TR1 at all. In the SDS gel, recombinant MARK2 appears at ∼88 kD (Fig. 3C, top: lanes 4-6), recombinant MARKK runs at 134 kD (lanes 7-9). In combination, MARKK shows the same Mr value, while that of MARK2 is shifted up to ∼97 kD due to phosphorylation by MARKK (lanes 1-3). In the autoradiogram (Fig. 3C, bottom), MARK2 reveals little autophosphorylation, indicating that this is not responsible for the basal activity of MARK2 (lanes 4-6). MARKK alone also shows only slight autophosphorylation (lanes 7-9; note that the 88 kD band is probably a degradation product of MARKK). By contrast, combining MARK2 and MARKK (lanes 1-3) leads to a strong incorporation of phosphate into MARK2, but only minor phosphorylation of MARKK. This confirms that MARKK can phosphorylate (and activate) MARK2, but the reverse does not hold.

### EXAMPLE 5

### Site-directed mutagenesis of MARK2 and its impact on the phosphorylating activity of MARKK

Site-directed mutagenesis was performed using the QuickChange kit (Stratagene) according to the instructions of the provider.

Previous experiments (DREWES, G. et al. 1997) revealed two phosphorylation sites in the activation loop of MARKs, T208 and S212 (Fig. 4A). When MARK was purified from brain, the removal of phosphates by PP-2a lead to inactivation, but it was not clear what the interplay between the sites was. This issue was addressed by site-directed mutagenesis.

Fig. 4B illustrates the activity of MARK2 carrying mutations in the activation loop or at K82, without or with activation by recombinant MARKK. Lanes 1 and 2 show the 10-fold activation by MARKK. Disturbing the ATP site by the K82R mutation destroys the activity (top, bars 3 & 4). When T208 was mutated into Ala, the activation by MARKK was lost and only basal activity remained (bars 5 & 6). This means that T208 is important for activation, and that the basal activity does not depend on it. By contrast, when S212 or both T208 and S212 were mutated into Ala, the intrinsic and the activatable activities of MARK2 were completely abolished (bars 7 - 10). When T208 was mutated into Glu, the basal activity increased 4-fold but no further activation by MARKK takes place. By changing S212 of MARK into Glu the basal activity and the activation by MARKK was lost, independently of whether T208 is mutated to Glu or not.

The activation of MARK2 and the roles of the phosphorylation sites turned out to be unexpectedly complex (Fig. 4B, Fig. 5A): Full activation requires only the phosphorylation of T208; mutation of T208 into A abolishes activity, and replacement by E mimics it partially (40%). MARKK does not phosphorylate S212 at all, so that the phosphorylation of this residue in brain-derived MARK must stem from another kinase. All mutations of S212 destroyed the activity of MARK, both S212A (designed for constitutive inactivation) and S212E (intended to increase activity). Even the phosphorylation at T208 was not able to override the negative effect of S212 mutations. Thus the presence of S212 is essential for the activation of MARK2, even though its phosphorylation is not and appears to be inhibitory. It is likely that the hydroxyl group of S212 is needed for stabilising the activation loop, as suggested by analogy with known kinases (Fig. 5A, B).

### EXAMPLE 6

### Use of antibodies for the verification of the ability of MARKK to activate MARK

To determine whether the mutation of S212 affects the ability of MARKK to phosphorylate MARK antibodies were raised against the regulatory peptide (phosphorylated specifically at T208 or S212). MARK2wt or the T208A- or S212A-mutants all become phosphorylated only in the presence of MARKK (as indicated by an Mr shift, Fig. 4C top, lanes 2, 4, 6). The pT208-specific antibody recognises MARK2wt and MARK2/S212A treated with MARKK (Fig. 4C bottom, lanes 2 and 6) but not phosphorylated MARK2/T208A (lane 4). This verifies that MARKK phosphorylates MARK2 at T208 even when the S212 is mutated to alanine. The phospho-S212-specific antibody could only detect MARK purified from brain but not recombinant MARK2 or its mutants showing that the S212 is not a target for MARKK (not shown). The data are summarised in Fig 5A: p-T208 plus S212 causes maximal activity, Thr or Ala at 208 plus S212 suffices for basal activity, and absence of S212 destroys the basal activity, even when T208 is phosphorylated (Fig. 4C, lane 6). These features can be explained in terms of known structures of kinases (Fig. 5B). The Mr shift of MARK2 is due to other sites phosphorylated by MARKK but unrelated to the activity of MARK2 (Fig. 4C, lane 5), and serves as an indicator for active MARKK.

### EXAMPLE 7

### Phosphoaminoacid analysis

The peptide ₂₀₃GNKLDTFCGSPPYAAPELQGKK₂₂₄ (SEQ ID NO: 4) corresponding to the regulatory lip of MARK2 was phosphorylated by MARKK for 3 h at 30°C to demonstrate the specificity of MARKK for T208 in the activation loop of MARK. An aliquot was partially hydrolysed in 6N HCl (110°C, 60 min) and analysed by one-dimensional thin-layer-electrophoresis at pH 2.5 (BOYLE, W.J. et al. 1991).

The peptide contains three phosphorylatable residues, but phosphate is incorporated almost exclusively at T208 (Fig. 6). Since S212 is followed by a proline it was considered whether a proline-directed kinase might contribute to the regulation of activity. Several kinases were tested (p38 stress activated kinase, p42/ERK2, p44/ERK1, Cdc2, Cdk5, GSK3 , CKI), but they had no effect on the basal activity of MARK or its activation by MARKK, and did not induce the antibody reaction against phospho-S212 (data not shown).

### EXAMPLE 8

### Co-immunoprecipitation analysis

8 × 10⁶ Sf9 cells were plated on T25 flask, infected with recombinant baculoviruses encoding HA-tagged MARK2 and myc-tagged MARKK kinases at a MOI of 1, and incubated at 27°C for 72 h. The cells were resuspended in 250 1 ice cold lysis buffer LB (50 mM Tris-HCl pH 7.4, 100 mM NaCl, 3 mM EGTA, 3 mM MgCl₂, 0.1% NP-40, 5 mM Chapso, 1 mM DTT, 2 mM benzamidin, 1 mM Na₃VO₄, 2 M MC_{LR} and Sigma mammalian protease inhibitor cocktail (final concentrations 0.8 M aprotinin, 20 M leupeptin, 40 M bestatin, 15 M pepstatin, 1 mM AEBSF and 14 M E-64) for 30 min, then centrifuged at 14000 rpm at 4°C. Agarose beads conjugated with anti-HA-antibody (Santa Cruz) were added to the supernatant (50 g/ml, 4°C, 4 h), then spun at 12000 rpm for 20 s. The pellet was washed with LB buffer without Chapso and resuspended in kinase assay buffer. 1 l of beads suspension was used for the kinase assay.

To show that MARKK stimulates MARK2 in cells Sf9 cells were transfected with baculovirus-encoded MARK2 or MARK2 plus MARKK. Cell extracts were immunoblotted to demonstrate the expression of MARK2 and MARKK (Fig. 7). Immunoprecipitation of the HA-tagged MARK2 from the cell lysates shows that co-expression of MARKK does not alter the level of MARK2. This provides a basis for comparing the MARK2 activity in Sf9 cells alone or with MARKK. Judging by the phosphorylation of the tau peptide TR1, MARK2 expressed in Sf9 cells becomes -3-fold more active when it is co-expressed with MARKK. Next it was tested whether MARK2 interacts directly with MARKK in Sf9 cells, using immunoprecipitation of transfected MARK2 with the HA-tag antibody. In the case of co-expression of HA-MARK2 with MARKK no detectable amounts of MARKK were found by immunoprecipitation. However, when the dominant negative mutant MARK2 (T208A/S212A) was co-expressed with MARKK both proteins were immunoprecipitated.

### EXAMPLE 9

### Cell culture and immunofluorescence

CHO wt cells and CHS cells stably transfected with hTau40 were grown in HAM's F12 medium with 10% FCS and incubated in a humidified atmosphere containing 5% CO2 at 37°C. For stable transfection G418 (600 g/ml, Invitrogen) was added to the culture medium. Cells were seeded at 70% confluence in 24-well plates on coverslips and transfected with DOTAP (Roche), followed by immunofluorescence analysis at various time points. Cells were washed in 80 mM Pipes, 1mM MgCl₂, 1 mM EGTA, 4% (w/v) polyethylene glycol (pH 6.9), fixed with 3.7% paraformaldehyde (Sigma) at room temperature, permeabilized with 80% methanol at -20°C for 5 min, incubated with primary antibody for 1 h at 37°C and treated with 10% goat serum in PBS for 10 min, then with labelled secondary antibody at 1:200 dilution for 1 h at 37°C. PC12 cells were grown in Dulbecco's modified Eagle's medium (DMEM) supplemented with 15% horse serum and 5% fetal bovine serum (FBS). For differentiation cells were treated with 100 ng/ml NGF in 0.1% FBS containing HAM's medium for 48 or 72 hrs. Confocal microscopy was done with a Zeiss LSM510 microscope using a 63 × objective.

It was shown earlier that overexpression of MARK leads to phosphorylation of MAPs at the KXGS motifs, their detachment from microtubules, and increased microtubule dynamics. In CHO cells this results in microtubule breakdown and cell death, which can be compensated by increasing MAPs and thus stabilising microtubules (DREWES, G. et al. 1997). In neuronal cells (N2a) the consequence of increased microtubule dynamics is the stimulation of neurite outgrowth (BIERNAT, J. et al. 2002).

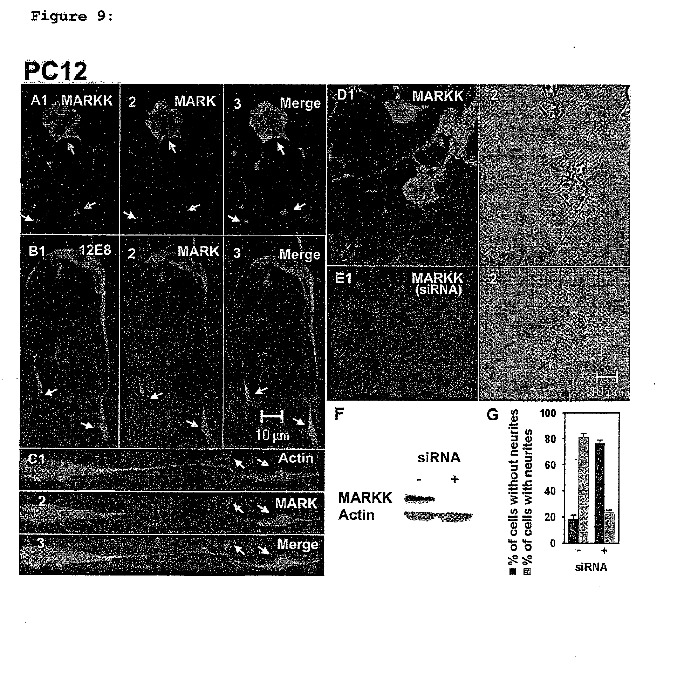

If MARKK indeed activated MARK one would expect similar consequences of MAP phosphorylation and increased microtubule dynamics when MARKK is overexpressed in cells. Fig. 8 and 9 illustrate the operation of the MARKK-MARK cascade in cells. One set of experiments were performed in CHO cells which contain endogenous MAP4 (phosphorylatable at KXGS motifs in the repeat domain). Fig. 8A shows CHO cells transfected with MARKK (labelled with YFP). While normal cells have an extended shape with a clear microtubule network, transfection by MARKK leads to loss of microtubules, shrinkage of the cells and eventually cell death (see Fig. 8A1, A2, arrows). As a control, transfection with inactive MARKK^{K57A} does not perturb microtubules, demonstrating that kinase activity is required for the effect (Fig. 8C). Microtubule perturbation is also observed when MARKK (YFP) and MARK (CFP) are co-transfected in CHO cells (Fig. 8B).

It was further shown that stabilisation of microtubules, for example by taxol, could prevent the catastrophic effect of MARKK. Fig. 8D shows that 10 M taxol prevents microtubule destruction and cell death. Note that MARKK is well expressed (Fig. 8D1, arrow), but the cell has a normal microtubule network and size (Fig. 8D2, arrow).

To verify the relationship between MARKK, MARK, MAPs and microtubules, CHO cells were stably transfected with htau40 in order to increase the stability of microtubules (analogous to the taxol experiment). Fig. 8E demonstrates that transfection of MARKK leads to expression of the kinase but cannot destroy the microtubules because they are stabilised by excess tau (Fig. 8E, arrows). Apparently the activity of the endogenous MARK is too low (also supported by the absence of tau staining by the 12E8 antibody).

A similar result is obtained when MARK was transiently transfected in the tau-stable CHO cells. Although there is now a higher concentration of active MARK (as seen by phospho-tau staining with 12E8 antibody, Fig. 8F2, arrow), the MARK activity still cannot counteract the stabilisation by tau, and the cell nearly retains its microtubule network. However, when the transfected MARK is constitutively active (mutant MARK^{T208E}), even the presence of tau cannot protect the microtubule network (Fig. 8G).

Similarly when the cells were co-transfected with MARKK and MARK the microtubule-stabilising effect of tau can be overcome by the phosphorylation of tau at the KXGS motifs (strong staining by 12E8 antibody, Fig. 8H3, arrow), and the detrimental effect of this phosphorylation is seen by the collapse of microtubules and the rounding up of cells (Fig. 8G, 8H). At the same time the antibody diagnostic for active MARK shows strong staining (Fig. 8I, arrow).

To verify that MARK plays an intermediary role in the cascade MARKK was co-transfected with an inactive MARK mutant (T208A, S212A) (Fig. 8J2, K2). As anticipated, tau shows no phosphorylation, the cells remain healthy (Fig. 8J3, J4, arrow), and there is no detectable activated MARK (Fig. 8K3).

### EXAMPLE 10

### RNAi preparation and transfections

21-nucleotide RNAs were designed as recommended (ELBASHIR, S.M. et al. 2001) and chemically synthesised by Qiagen-Science (Germantown, MD). The RNAi targeting sequences of MARKK/TAO-1 corresponded to the coding regions 182-202 and 1699-1719.

Transfections of RNAi for endogenous MARKK/TAO1 gene silencing were carried out with Oligofectamine (Invitrogen) using simultaneously both RNAi complexes at final concentrations of 100 nM. 24 hrs after transfection cells were differentiated for 24-48 hrs with 100 ng/ml NGF before IF analysis or Western blots were performed. Neurite extension was quantified by analysing 3 experiments with 200 cells each.

In another set of experiments the expression of endogenous MARKK, MARK, and the phosphorylation of tau in PC12 cells differentiated with NGF was studied (Fig. 9). In contrast to CHO cells, neuronal cells (PC12) have higher levels of endogenous MARKK and MARK which can be seen by immunostaining (Fig. 9). This allows observing the endogenous functions of the kinases without the risk of artefacts due to overexpression.

Previous studies revealed that transfection of MARK induced tau phosphorylation concomitant with neurite outgrowth, and that MARK, activated MARK, phospho-KXGS-tau and actin co-localised (BIERNAT, J. et al. 2002). The effect was abolished if the KXGS motifs of tau were mutated into KXGA, or if a dominant negative mutant of MARK was expressed.

Fig. 9 illustrates PC12 cells differentiated with 100 ng/ml NGF and then immunostained for endogenous MARKK and MARK. Differentiation causes upregulation of MARK (and to a lesser extent MARKK, data not shown), and both kinases co-localize - together with phospho-tau and the actin network - near the plasma membrane and at the growth cones (Fig. 9A, B, C).

As a control, the ability of MARKK to promote neurite outgrowth after the NGF trigger can be suppressed by downregulating MARKK with RNAi (Fig. 9E, F). While mock-transfected PC12 cells can be differentiated with high efficiency (Fig. 9D, G), those transfected with MARKK-RNAi show only negligible amounts of endogenous MARKK and their differentiation is suppressed (20%, Fig. 9E, G). The result is corroborated by Western blotting showing that after MARKK-RNAi transfection into PC12 cells the protein was suppressed (Fig. 9F). Thus, all experiments are consistent with a cascade connecting MARKK, MARK, tau (or related MAPS), microtubule plasticity, and cell shape.

### REFERENCES

ANOSTARIO, M., HARRISON, M.L. and GEAHLEN, R.L. (1990) Immunochemical detection of adenine nucleotide-binding proteins with antibodies to 5'-p-fluorosulfonylbenzoyladenosine. Analyt. Biochem. **190:** 60-65.

BAEKELANDT, V., DE STROOPER, B., NUTTIN, B. and DEBYSER, Z. (2000) Gene therapeutic strategies for neurodegenerative diseases. Curr. Opin. Mol. Ther. **2(5)**:540-554.

BIERNAT, J., WU, Y.Z., TIMM, T., ZHENG-FISCHHÖFER, Q., MANDELKOW, E., MEIJER, L. and MANDELKOW, E.-M. (2002) Protein kinase MARK/PAR-1 is required for neurite outgrowth and establishment of neuronal polarity. Mol. Biol. Cell. **13:** 4013-4028.

BÖHM, H., BRINKMANN, V., DRAB, M. , HENSKE, A. and KURZCHALIA, T.V. (1997) Mammalian homologues of C. elegans PAR-1 are asymmetrically localized in epithelial cells and may influence their polarity. Curr. Biol. **7:** 603-606.

BOYLE, W.J., VAN DER GEER, P. and HUNTER, T. (1991). Phosphopeptide mapping and phosphoamino acid analysis by two-dimensional separation on thin layer cellulose plates. Methods Enzymol. **201**: 110-149.

CHEN, Z., HUTCHISON, M. and COBB, M.H. (1999) Isolation of the protein kinase TAO2 and identification of its mitogen-activated protein kinase/extracellular signal-regulated kinase kinase binding domain. J. Biol. Chem. **274:** 28803-28807.

CHEN, Z., GIBSON, T.B., ROBINSON, F., SILVESTRO, L., PEARSON, G., XU BE, B.E., WRIGHT, A., VANDERBILT, C. and Cobb, M.H. (2001) MAP Kinases. Chem. Rev. **101:** 2449-2476.

DAN, I., WATANABE, N.M. and KUSUMI, A. (2001) The Ste20 group kinases as regulators of MAP kinase cascades. Trends Cell. Biol. **11**: 220-230.

DREWES, G., EBNETH, A., PREUSS, U., MANDELKOW, E. -M. and MANDELKOW, E. (1997) MARK - a novel family of protein kinases that phosphorylate microtubule-associated proteins and trigger microtubule disruption. Cell **89**: 297-308.

EBNETH, A. , GODEMANN, R. , STAMER, K. , ILLENBERGER, S. , TRINCZEK, B. and MANDELKOW, E. (1998) Overexpression of tau protein inhibits kinesin-dependent trafficking of vesicles, mitochondria, and endoplasmic reticulum: implications for Alzheimer's disease. J. Cell Biol. **143(3):** 777-794.

EBNETH, A., DREWES, G., MANDELKOW, E.M. and MANDELKOW, E. (1999) Phosphorylation of MAP2c and MAP4 by MARK kinases leads to destabilization of microtubules in cells. Cell Motil. Cytoskeleton **44 (3)** : 209-224.

ELBASHIR, S.M., HARBORTH, J., LENDECKEL, W., YALCIN, A., WEBER, K. and TUSCHL, T. (2001). Duplexes of 21-nucleotide RNAs mediate RNA interference in cultured mammalian cells. Nature **411**: 494- 498.

FOLSTEIN, M.F., FOLSTEIN, S.E. and MCHUGH P.R. (1975) "Mini-mental state". A practical method for grading the cognitive state of patients for the clinician. J. Psychiatr. Res. **12(3)**: 189-198.

GEVAERT, K., DEMOL, H., MARTENS, L., HOORELBEKE, B., PUYPE, M., GOETHALS, M., VAN DAMME, J., DE BOECK, S. and VANDEKERCKHOVE, J. (2001) Protein identification based on matrix assisted laser desorption/ionization-post source decay-mass spectrometry. Electrophoresis **22:** 1645-1651.

GOEDERT, M., WISCHIK, C.M., CROWTHER, R.A., WALKER, J.E. and KLUG, A. (1988) Cloning and sequencing of the cDNA encoding a core protein of the paired helical filament of Alzheimer disease: identification as the microtubule-associated protein tau. Proc Natl Acad Sci U S A. **85(11):**4051-4055.

GOTZ, J., CHEN, F., VAN DORPE, J. and NITSCH, R.M. (2001) Formation of neurofibrillary tangles in P3011 tau transgenic mice induced by Abeta 42 fibrils. Science **293(5534):** 1491-1495.

Guo, S. and KEMPHUES, K.J. (1995) Par-1, a gene required for establishing polarity in C. elegans embryos, encodes a putative Ser/Thr kinase that is asymmetrically distributed. Cell **81**: 611-620.

HANKS, S.K. and HUNTER, T. (1995) Protein kinases 6. The eukaryotic protein kinase superfamily: kinase catalytic domain structure and classification. FASEB J. **9**: 576-596.

HUTCHISON, M., BERMAN, K.S. and COBB, M.H. (1998) Isolation of TAO1, a protein kinase that activates MEKs in stress-activated protein kinase cascades. J. Biol. Chem. **273**: 28625-28632.

IQBAL, K. and GRUNDKE-IQBAL, I. (1997) Elevated levels of τ and ubiquitin in brain and cerebrospinal fluid in Alzheimer's disease. Int. Psychogeriatr. **9** (Suppl. 1): 289-296.

JOHNSON, L.N., NOBLE, M.E.M. and OWEN, D.J. (1996) Active and inactive protein kinases: Structural basis for regulation. Cell **85**: 149-158.

KASPAR, B.K., LLADO, J., SHERKAT, N., ROTHSTEIN, J.D. and GAGE, F,H, (2003) Retrograde viral delivery of IGF-1 prolongs survival in a mouse ALS model. Science **301(5634):** 839-42.

LEWIS, J., MCGOWAN, E., ROCKWOOD, J., MELROSE, H., NACHARAJU, P., VAN SLEGTENHORST, M. , GWINN-HARDY, K. , PAUL MURPHY, M. , BAKER, M. , YU, X., DUFF, K., HARDY, J., CORRAL, A., LIN, W.L., YEN, S.H., DICKSON, D.W., DAVIES, P. and HUTTON, M. (2000) Neurofibrillary tangles, amyotrophy and progressive motor disturbance in mice expressing mutant (P301L) tau protein. Nat Genet. **25(4)**: 402-405.

MANNING, G., WHYTE, D.B., MARTINEZ, R., HUNTER, T. and SUDARSANAM, S. (2002) The protein kinase complement of the human genome. Science **298:** 1912-1934.

PARSA, I. (1988) Loss of a Mr 78,000 marker in chemically induced transplantable carcinomas and primary carcinoma of human pancreas. Cancer Res. **48:** 2265-2272.

SCOTT, J.W., NORMAN, D.G., HAWLEY, S.A., KONTOGIANNIS, L. and HARDIE, D.G. (2002) Protein kinase substrate recognition studied using the recombinant catalytic domain of AMP-activated protein kinase and a model substrate. J. Mol. Biol. **317:** 309-323.

SEUBERT, P., MAWAL-DEWAN, M., BARBOUR, R., JAKES, R., GOEDERT, M., JOHNSON, G.V., LITERSKY, J.M., SCHENK, D., LIEBERBURG, I. and TROJANOWSKI, J.Q. (1995) Detection of phosphorylated Ser262 in fetal tau, adult tau and paired helical filament tau. J. Biol. Chem. 270(32): 18917-19822.

SHELEG, S.V., KOROTKEVICH, E.A., ZHAVRID, E.A., MURAVSKAYA, G.V., SMEYANOVICH, A.F., SHANKO, Y.G., YURKSHTOVICH, T.L., BYCHKOVSKY, B. and BELYAEV, S.A. (2002) Local chemotherapy with cisplatin-depot for glioblastoma multiforme. J. Neurooncol. **60(1):** 53-59.

SHULMAN, J.M., BENTON, R. and ST. JOHNSTON, D. (2000) The Drosophila homolog of C. elegans PAR-1 organizes the oocyte cytoskeleton and directs oskar mRNA localization to the posterior pole. Cell **101**: 377-388.

STAMER, K, VOGEL, R., THIES, E., MANDELKOW, E. and MANDELKOW, E.M. (2002) Tau blocks traffic of organelles, neurofilaments, and APP vesicles in neurons and enhances oxidative stress. J. Cell Biol. **156(6):** 1051-1063.

TAMURA, K. and FUKUOKA, M. (2003) Molecular target-based cancer therapy: tyrosine kinase inhibitors. Int. J. Clin. Oncol. **8(4):** 207-211.

TASSI, E., BIESOVA, Z., DI FIORE, P.P., GUTKIND, J.S. and WONG, W.T. (1999) Human JIK, a novel member of the STE20 kinase family that inhibits JNK and is negatively regulated by epidermal growth factor. J. Biol. Chem. **274:** 33287-33295.

TIMM, T., LI, X.-Y., BIERNAT, J., JIAO, J., MANDELKOW, E., VANDERKERCKHOVE, J. and MANDELKOW, E.-M. (2003) MARKK, a Ste20-like kinase, activates the polarity-inducing kinase MARK/PAR-1. Gene (in press)

TOMANCAK, P., PIANO, F., RIECHMANN, V., GUNSALUS, K.C., KEMPHUES, K.J. and EPHRUSSI, A. (2000) A Drosophila melanogaster homologue of Caenorhabditis elegans par-1 acts at an early step in embryonic-axis formation. Nat. Cell. Biol. **2:** 458-460.

VANDERMEEREN, M., MERCKEN, M., VANMECHELEN, E., SIX, J., VAN DE VOORDE, A., MARTIN, J.J. and CRAS. P. (1993) Detection of tau proteins in normal and Alzheimer's disease cerebrospinal fluid with a sensitive sandwich enzyme-linked immunosorbent assay. J. Neurochem. **61(5)**: 1828-1834.

VOGELSBERG-RAGAGLIA, V., BRUCE, J., RICHTER-LANDSBERG, C., ZHANG, B., HONG, M., TROJANOWSKI, J.Q. and LEE, V.M. (2000) Distinct FTDP-17 missense mutations in tau produce tau aggregates and other pathological phenotypes in transfected CHO cells. Mol. Biol. Cell **11(12)**: 4093-104.

VOGELSBERG-RAGAGLIA, M., ISHIHARA, T., ZHANG, B., HONG, M., ANDREADIS, A., TROJANOWSKI, J. and LEE, V.M. (2002) Transgenic mouse model of tauopathies with glial pathology and nervous system degeneration. Neuron. **35(3):** 433-46.

## Claims

1. Use of MARKK (microtubule affinity regulating kinase kinase) or MARKK antagonists for the preparation of a pharmaceutical composition for the treatment of pathologies **characterised by** increased or reduced phosphorylation of MARK or tau protein at KXGS sites.

2. Use according to claim 1, **characterised in that** the increased or reduced phosphorylation is present in cells of a mammal.

3. Use according to claim 2, **characterised in that** the mammal is a human.

4. Use according to any of claims 1 to 3, **characterised in that** the pathology is associated with increased phosphorylation of tau protein resulting in less than 20% tau protein of total tau bound to microtubules.

5. Use according to any of claims 1 to 3, **characterised in that** the pathology is associated with a reduced phosphorylation of tau protein resulting in at least one to two times more tau protein bound to microtubules compared to healthy cells.

6. Use according to any of claims 1 to 5, **characterised in that** MARKK comprises the amino acid sequence provided in SEQ ID NO: 3, is a homologue, derivative or variant thereof that is at least 80% identical to the amino acid sequence provided in SEQ ID NO: 3, or is a fragment thereof wherein the ability to phosphorylate MARK is not substantially diminished.

7. Use according to any of claims 1 to 6, **characterised in that** the MARKK antagonist is selected from the group consisting of an antibody, an antisense construct, a siRNA, a peptide that specifically binds to MARKK, an agent or an enzyme, that is capable of substantially inhibiting the phosphorylating activity of MARKK, and a phosphatase.

8. Use according to claim 7, **characterised in that** the agent that is capable of substantially inhibiting the phosphorylating activity of MARKK is PAK5.

9. Use according to claim 7, **characterised in that** the MARKK antagonist is an antibody.

10. Use according to any of claims 1 to 9, wherein the pharmaceutical composition is administered orally or parenterally.

11. Use according to any of claims 1 to 10, wherein the pharmaceutical composition is administered as part of a sustained release formulation administered by depot implantation.

12. Use according to any of claims 1 to 11, wherein the enzyme, peptide, agent or antibody capable of substantially inhibiting the phosphorylating activity of MARKK is a MARKK antagonists administered using genetically manipulated viruses selected from the group consisting of Adenoviruses and Lentiviruses deficient in reproduction coding for the respective MARKK antagonist.

13. Use according to any of claims 1 to 12, **characterised in that** the increased or reduced phosphorylation is present in neurons of a mammal.

14. Use according to claim 13, **characterised in that** the mammal is a human.

15. Use according to any of claims 1 to 14, **characterised in that** the pathology is Alzheimer's disease.

16. Use according to any of claims 1 to 15, **characterised in that** MARKK is used for the preparation of a pharmaceutical composition for the treatment of Alzheimer's disease, wherein the treatment comprises the phosphorylation of MARK or tau protein at KXGS sites.

17. Use according to any of claims 1 to 4 and 6 to 16, **characterised in that** the pathology is associated with increased phosphorylation of tau protein resulting in less than 20 % tau proteins of total tau bound to microtubules is selected from the group of Tauopathies consisting of CBD (Cortical Basal Disease), PSP (Progressive Supra Nuclear Palsy), Parkinsonism, FTDP-17 (Fronto-Temporal Dementia with Parkinsonism linked to chromosome 17), Familiar British Dementia, Prion Disease (Creutzfeld Jakob Disease) and Pick's Disease.

18. Use according to any of claims 1 to 3 and 5 to 16, **characterised in that** the pathology is associated with reduced phosphorylation of tau protein resulting in more than one to two times more tau protein bound to microtubules compared to healthy neurons is selected from the group of Tauopathies consisting of CBD (Cortical Basal Disease), PSP (Progressive Supra Nuclear Palsy), Parkinsonism, FTDP-17 (Fronto-Temporal Dementia with Parkinsonism linked to chromosome 17), Familiar British Dementia, Prion Disease (Creutzfeld Jakob Disease) and Pick's Disease.

19. Use according to any of claims 1 to 12, **characterised in that** the pathology is cancer.


SEQUENCE LISTING

<110> Max-Planck Gesellschaft zur Förderung der Wissenschaft e.V.

<120> Use of MARKK or MARKK antagonists for the treatment of pathologies
characterised by increased or reduced phosphorylation of MARK or tau protein

<130> P642821

<140> EP 03400053.9
<141> 2003-09-15

<160> 8

<170> PatentIn version 3.1

<210> 1
<211> 13
<212> PRT
<213> Artificial Sequence

<220>
<223> synthetic peptide
<220>
<221> MISC_FEATURE
<222> (1)..(13)
<223> peptide derived from the first repeat of tau protein containing S
262 in the KXGS motif

<400> 1
Asn Val Lys Ser Lys Ile Gly Ser Thr Glu Asn Leu Lys
1               5                   10

<210> 2
<211> 730
<212> PRT
<213> Homo sapiens

<400>  2

Met Ser Ser Ala Arg Thr Pro Leu Pro Thr Leu Asn Glu Arg Asp Thr
1               5                   10                  15

Glu Gln Pro Thr Leu Gly His Leu Asp Ser Lys Pro Ser Ser Lys Ser
            20                  25                  30

Asn Met Leu Arg Gly Arg Asn Ser Ala Thr Ser Ala Asp Glu Gln Pro
        35                  40                  45

His Ile Gly Asn Tyr Arg Leu Leu Lys Thr Ile Gly Lys Gly Asn Phe
    50                  55                  60

Ala Lys Val Lys Leu Ala Arg His Ile Leu Thr Gly Lys Glu Val Ala
65                  70                  75                  80

Val Lys Ile Ile Asp Lys Thr Gln Leu Asn Ser Ser Ser Leu Gln Lys
                85                  90                  95

Leu Phe Arg Glu Val Arg Ile Met Lys Val Leu Asn His Pro Asn Ile
            100                 105                 110

Val Lys Leu Phe Glu Val Ile Glu Thr Glu Lys Thr Leu Tyr Leu Val
        115                 120                 125

Met Glu Tyr Ala Ser Gly Gly Glu Val Phe Asp Tyr Leu Val Ala His
    130                 135                 140

Gly Arg Met Lys Glu Lys Glu Ala Arg Ala Lys Phe Arg Gln Ile Val
145                 150                 155                 160

Ser Ala Val Gln Tyr Cys His Gln Lys Phe Ile Val His Arg Asp Leu
                165                 170                 175

Lys Ala Glu Asn Leu Leu Leu Asp Ala Asp Met Asn Ile Lys Ile Ala
            180                 185                 190

Asp Phe Gly Phe Ser Asn Glu Phe Thr Phe Gly Asn Lys Leu Asp Thr
        195                 200                 205

Phe Cys Gly Ser Pro Pro Tyr Ala Ala Pro Glu Leu Phe Gln Gly Lys
    210                 215                 220

Lys Tyr Asp Gly Pro Glu Val Asp Val Trp Ser Leu Gly Val Ile Leu
225                 230                 235                 240

Tyr Thr Leu Val Ser Gly Ser Leu Pro Phe Asp Gly Gln Asn Leu Lys
                245                 250                 255

Glu Leu Arg Glu Arg Val Leu Arg Gly Lys Tyr Arg Ile Pro Phe Tyr
            260                 265                 270

Met Ser Thr Asp Cys Glu Asn Leu Leu Lys Lys Phe Leu Ile Leu Asn
        275                 280                 285

Pro Ser Lys Arg Gly Thr Leu Glu Gln Ile Met Lys Asp Arg Trp Met
    290                 295                 300

Asn Val Gly His Glu Asp Asp Glu Leu Lys Pro Tyr Val Glu Pro Leu
305                 310                 315                 320

Pro Asp Tyr Lys Asp Pro Arg Arg Thr Glu Leu Met Val Ser Met Gly
                325                 330                 335

Tyr Thr Arg Glu Glu Ile Gln Asp Ser Leu Val Gly Gln Arg Tyr Asn
            340                 345                 350

Glu Val Met Ala Thr Tyr Leu Leu Leu Gly Tyr Lys Ser Ser Glu Leu
        355                 360                 365

Glu Gly Asp Thr Ile Thr Leu Lys Pro Arg Pro Ser Ala Asp Leu Thr
    370                 375                 380

Asn Ser Ser Ala Pro Ser Pro Ser His Lys Val Gln Arg Ser Val Ser
385                 390                 395                 400

Ala Asn Pro Lys Gln Arg Arg Ser Ser Asp Gln Ala Val Pro Ala Ile
                405                 410                 415

Pro Thr Ser Asn Ser Tyr Ser Lys Lys Thr Gln Ser Asn Asn Ala Glu
            420                 425                 430

Asn Lys Arg Pro Glu Glu Glu Thr Gly Arg Lys Ala Ser Ser Thr Ala
        435                 440                 445

Lys Val Pro Ala Ser Pro Leu Pro Gly Leu Asp Arg Lys Lys Thr Thr
    450                 455                 460

Pro Thr Pro Ser Thr Asn Ser Val Leu Ser Thr Ser Thr Asn Arg Ser
465                 470                 475                 480

Arg Asn Ser Pro Leu Leu Asp Arg Ala Ser Leu Gly Gln Ala Ser Ile
                485                 490                 495

Gln Asn Gly Lys Asp Ser Thr Ala Pro Gln Arg Val Pro Val Ala Ser
            500                 505                 510

Pro Ser Ala His Asn Ile Ser Ser Ser Ser Gly Ala Pro Asp Arg Thr
        515                 520                 525

Asn Phe Pro Arg Gly Val Ser Ser Arg Ser Thr Phe His Ala Gly Gln

530                 535                 540

Leu Arg Gln Val Arg Asp Gln Gln Asn Leu Pro Phe Gly Val Thr Pro
545                 550                 555                 560

Ala Ser Pro Ser Gly His Ser Gln Gly Arg Arg Gly Ala Ser Gly Ser
                565                 570                 575

Ile Phe Ser Lys Phe Thr Ser Lys Phe Val Arg Arg Asn Leu Asn Glu
            580                 585                 590

Pro Glu Ser Lys Asp Arg Val Glu Thr Leu Arg Pro His Val Val Gly
        595                 600                 605

Gly Gly Gly Thr Asp Lys Glu Lys Glu Glu Phe Arg Glu Ala Lys Pro
    610                 615                 620

Arg Ser Leu Arg Phe Thr Trp Ser Met Lys Thr Thr Ser Ser Met Glu
625                 630                 635                 640

Pro Asn Glu Met Met Arg Glu Ile Arg Lys Val Leu Asp Ala Asn Ser
                645                 650                 655

Cys Gln Ser Glu Leu His Glu Arg Tyr Met Leu Leu Cys Val His Gly
            660                 665                 670

Thr Pro Gly His Glu Asn Phe Val Gln Trp Glu Met Glu Val Cys Lys
        675                 680                 685

Leu Pro Arg Leu Ser Leu Asn Gly Val Arg Phe Lys Arg Ile Ser Gly
    690                 695                 700

Thr Ser Met Ala Phe Lys Asn Ile Ala Ser Lys Ile Ala Asn Glu Leu
705                 710                 715                 720

Lys Leu His His His His His His His His
                725                 730

<210>  3

<211>  1022

<212>  PRT

<213>  Homo sapiens

<220>

<221>  MISC_FEATURE

<222>  (1)..(1022)

<223>  his10-tagged at the N-terminus

<400> 3

Met Gly His His His His His His His His His His Ser Ser Gly His
1               5                   10                  15

Ile Glu Gly Arg His Met Pro Ser Thr Asn Arg Ala Gly Ser Leu Lys
            20                  25                  30

Asp Pro Glu Ile Ala Glu Leu Phe Phe Lys Glu Asp Pro Glu Lys Leu
        35                  40                  45

Phe Thr Asp Leu Arg Glu Ile Gly His Gly Ser Phe Gly Ala Val Tyr
    50                  55                  60

Phe Ala Arg Asp Val Arg Thr Asn Glu Val Val Ala Ile Lys Lys Met
65                  70                  75                  80

Ser Tyr Ser Gly Lys Gln Ser Thr Glu Lys Trp Gln Asp Ile Ile Lys
                85                  90                  95

Glu Val Lys Phe Leu Gln Arg Ile Lys His Pro Asn Ser Ile Glu Tyr
            100                 105                 110

Lys Gly Cys Tyr Leu Arg Glu His Thr Ala Trp Leu Val Met Glu Tyr
        115                 120                 125

Cys Leu Gly Ser Ala Ser Asp Leu Leu Glu Val His Lys Lys Pro Leu
    130                 135                 140

Gln Glu Val Glu Ile Ala Ala Ile Thr His Gly Ala Leu Gln Gly Leu
145                 150                 155                 160

Ala Tyr Leu His Ser His Thr Met Ile His Arg Asp Ile Lys Ala Gly
                165                 170                 175

Asn Ile Leu Leu Thr Glu Pro Gly Gln Val Lys Leu Ala Asp Phe Gly
            180                 185                 190

Ser Ala Ser Met Ala Ser Pro Ala Asn Ser Phe Val Gly Thr Pro Tyr
        195                 200                 205

Trp Met Ala Pro Glu Val Ile Leu Ala Met Asp Glu Gly Gln Tyr Asp
    210                 215                 220

Gly Lys Val Asp Val Trp Ser Leu Gly Ile Thr Cys Ile Glu Leu Ala
225                 230                 235                 240

Glu Arg Lys Pro Pro Leu Phe Asn Met Asn Ala Met Ser Ala Leu Tyr
                245                 250                 255

His Ile Ala Gln Asn Glu Ser Pro Thr Leu Gln Ser Asn Glu Trp Ser

            260                 265                 270

Asp Tyr Phe Arg Asn Phe Val Asp Ser Cys Leu Gln Lys Ile Pro Gln
        275                 280                 285

Asp Arg Pro Thr Ser Glu Glu Leu Leu Lys His Met Phe Val Leu Arg
    290                 295                 300

Glu Arg Pro Glu Thr Val Leu Ile Asp Leu Ile Gln Arg Thr Lys Asp
305                 310                 315                 320

Ala Val Arg Glu Leu Asp Asn Leu Gln Tyr Arg Lys Met Lys Lys Leu
                325                 330                 335

Leu Phe Gln Glu Ala His Asn Gly Pro Ala Val Glu Ala Gln Glu Glu
            340                 345                 350

Glu Glu Glu Gln Asp His Gly Gly Gly Arg Thr Gly Thr Val Asn Ser
        355                 360                 365

Val Gly Ser Asn Gln Ser Ile Pro Ser Met Ser Ile Ser Ala Ser Ser
    370                 375                 380

Gln Ser Ser Ser Val Asn Ser Leu Pro Asp Ala Ser Asp Asp Lys Ser
385                 390                 395                 400

Glu Leu Asp Met Met Glu Gly Asp His Thr Val Met Ser Asn Ser Ser
                405                 410                 415

Val Ile His Leu Lys Pro Glu Glu Glu Asn Tyr Gln Glu Glu Gly Asp
            420                 425                 430

Pro Arg Thr Arg Ala Ser Asp Pro Gln Ser Pro Pro Gln Val Ser Arg
        435                 440                 445

His Lys Ser His Tyr Arg Asn Arg Glu His Phe Ala Thr Ile Arg Thr
    450                 455                 460

Ala Ser Leu Val Thr Arg Gln Met Gln Glu His Glu Gln Asp Ser Glu
465                 470                 475                 480

Leu Arg Glu Gln Met Ser Gly Tyr Lys Arg Met Arg Arg Gln His Gln
                485                 490                 495

Lys Gln Leu Met Thr Leu Glu Asn Lys Leu Lys Ala Glu Met Asp Glu
            500                 505                 510

His Arg Leu Arg Leu Asp Lys Asp Leu Glu Thr Gln Arg Asn Asn Phe
        515                 520                 525

Ala Ala Glu Met Glu Lys Leu Ile Lys Lys His Gln Ala Ser Met Glu
    530                 535                 540

Lys Glu Ala Lys Val Met Ala Asn Glu Glu Lys Lys Phe Gln Gln His
545                 550                 555                 560

Ile Gln Ala Gln Gln Lys Lys Glu Leu Asn Ser Phe Leu Glu Ser Gln
                565                 570                 575

Lys Arg Glu Tyr Lys Leu Arg Lys Glu Gln Leu Lys Glu Glu Leu Asn
            580                 585                 590

Glu Asn Gln Ser Thr Pro Lys Lys Glu Lys Gln Glu Trp Leu Ser Lys
        595                 600                 605

Gln Lys Glu Asn Ile Gln His Phe Gln Ala Glu Glu Glu Ala Asn Leu
    610                 615                 620

Leu Arg Arg Gln Arg Gln Tyr Leu Glu Leu Glu Cys Arg Arg Phe Lys
625                 630                 635                 640

Arg Arg Met Leu Leu Gly Arg His Asn Leu Glu Gln Asp Leu Val Arg
                645                 650                 655

Glu Glu Leu Asn Lys Arg Gln Thr Gln Lys Asp Leu Glu His Ala Met
            660                 665                 670

Leu Leu Arg Gln His Glu Ser Met Gln Glu Leu Glu Phe Arg His Leu
        675                 680                 685

Asn Thr Ile Gln Lys Met Arg Cys Glu Leu Ile Arg Leu Gln His Gln
    690                 695                 700

Thr Glu Leu Thr Asn Gln Leu Glu Tyr Asn Lys Arg Arg Glu Arg Glu
705                 710                 715                 720

Leu Arg Arg Lys His Val Met Glu Val Arg Gln Gln Pro Lys Ser Leu
                725                 730                 735

Lys Ser Lys Glu Leu Gln Ile Lys Lys Gln Phe Gln Asp Thr Cys Lys
            740                 745                 750

Ile Gln Thr Arg Gln Tyr Lys Ala Leu Arg Asn His Leu Leu Glu Thr
        755                 760                 765

Thr Pro Lys Ser Glu His Lys Ala Val Leu Lys Arg Leu Lys Glu Glu
    770                 775                 780

Gln Thr Arg Lys Leu Ala Ile Leu Ala Glu Gln Tyr Asp His Ser Ile
785                 790                 795                 800

Asn Glu Met Leu Ser Thr Gln Ala Leu Arg Leu Asp Glu Ala Gln Glu
                805                 810                 815

Ala Glu Cys Gln Val Leu Lys Met Gln Leu Gln Gln Glu Leu Glu Leu
            820                 825                 830

Leu Asn Ala Tyr Gln Ser Lys Ile Lys Met Gln Ala Glu Ala Gln His
        835                 840                 845

Asp Arg Glu Leu Arg Glu Leu Glu Gln Arg Val Ser Leu Arg Arg Ala
    850                 855                 860

Leu Leu Glu Gln Lys Ile Glu Glu Glu Met Leu Ala Leu Gln Asn Glu
865                 870                 875                 880

Arg Thr Glu Arg Ile Arg Ser Leu Leu Glu Arg Gln Ala Arg Glu Ile
                885                 890                 895

Glu Ala Phe Asp Ser Glu Ser Met Arg Leu Gly Phe Ser Asn Met Val
            900                 905                 910

Leu Ser Asn Leu Ser Pro Glu Ala Phe Ser His Ser Tyr Pro Gly Ala
        915                 920                 925

Ser Ser Trp Ser His Asn Pro Thr Gly Gly Ser Gly Pro His Trp Gly
    930                 935                 940

His Pro Met Gly Gly Thr Pro Gln Ala Trp Gly His Pro Met Gln Gly
945                 950                 955                 960

Gly Pro Gln Pro Trp Gly His Pro Ser Gly Pro Met Gln Gly Val Pro
                965                 970                 975

Arg Gly Ser Ser Ile Gly Val Arg Asn Ser Pro Gln Ala Leu Arg Arg
            980                 985                 990

Thr Ala Ser Gly Gly Arg Thr Glu  Gln Gly Met Ser Arg  Ser Thr Ser
        995                 1000                 1005

Val Thr  Ser Gln Ile Ser Asn  Gly Ser His Met Ser  Tyr Thr
    1010                 1015                 1020

<210>  4

<211>  22

<212>  PRT

<213>  Artificial Sequence


<220>

<223>  synthetic peptide

<220>

<221>  PEPTIDE

<222>  (1)..(22)

<223>  peptide represents residues 203 to 224 from the activation loop o
       f MARK2 and contains three phosphorylatable residues

<400>  4

Gly Asn Lys Leu Asp Thr Phe Cys Gly Ser Pro Pro Tyr Ala Ala Pro
1               5                   10                  15

Glu Leu Gln Gly Lys Lys
            20

<210>  5

<211>  403

<212>  PRT

<213>  Homo sapiens

<220>

<221>  MISC_FEATURE

<222>  (1)..(403)

<223>  contains residues 1-368 of MARK and a N-terminal His12-tag; T208
       and S212 in the regulatory loop are mutated to alanine

<400>  5

Met Gly His His His His His His His His His His Ser Ser Gly His
1               5                   10                  15

Ile Glu Gly Arg His Met Gly Ser Tyr Pro Tyr Asp Val Pro Asp Tyr
            20                  25                  30

Ala Ser Asp Met Gly Ala Met Ser Ser Ala Arg Thr Pro Leu Pro Thr
        35                  40                  45

Leu Asn Glu Arg Asp Thr Glu Gln Pro Thr Leu Gly His Leu Asp Ser
    50                  55                  60

Lys Pro Ser Ser Lys Ser Asn Met Leu Arg Gly Arg Asn Ser Ala Thr
65                  70                  75                  80

Ser Ala Asp Glu Gln Pro His Ile Gly Asn Tyr Arg Leu Leu Lys Thr
                85                  90                  95

Ile Gly Lys Gly Asn Phe Ala Lys Val Lys Leu Ala Arg His Ile Leu
            100                 105                 110

Thr Gly Lys Glu Val Ala Val Lys Ile Ile Asp Lys Thr Gln Leu Asn
        115                 120                 125

Ser Ser Ser Leu Gln Lys Leu Phe Arg Glu Val Arg Ile Met Lys Val
    130                 135                 140

Leu Asn His Pro Asn Ile Val Lys Leu Phe Glu Val Ile Glu Thr Glu
145                 150                 155                 160

Lys Thr Leu Tyr Leu Val Met Glu Tyr Ala Ser Gly Gly Glu Val Phe
                165                 170                 175

Asp Tyr Leu Val Ala His Gly Arg Met Lys Glu Lys Glu Ala Arg Ala
            180                 185                 190

Lys Phe Arg Gln Ile Val Ser Ala Val Gln Tyr Cys His Gln Lys Phe
        195                 200                 205

Ile Val His Arg Asp Leu Lys Ala Glu Asn Leu Leu Leu Asp Ala Asp
    210                 215                 220

Met Asn Ile Lys Ile Ala Asp Phe Gly Phe Ser Asn Glu Phe Thr Phe
225                 230                 235                 240

Gly Asn Lys Leu Asp Ala Phe Cys Gly Ala Pro Pro Tyr Ala Ala Pro
                245                 250                 255

Glu Leu Phe Gln Gly Lys Lys Tyr Asp Gly Pro Glu Val Asp Val Trp
            260                 265                 270

Ser Leu Gly Val Ile Leu Tyr Thr Leu Val Ser Gly Ser Leu Pro Phe
        275                 280                 285

Asp Gly Gln Asn Leu Lys Glu Leu Arg Glu Arg Val Leu Arg Gly Lys
    290                 295                 300

Tyr Arg Ile Pro Phe Tyr Met Ser Thr Asp Cys Glu Asn Leu Leu Lys
305                 310                 315                 320

Lys Phe Leu Ile Leu Asn Pro Ser Lys Arg Gly Thr Leu Glu Gln Ile
                325                 330                 335

Met Lys Asp Arg Trp Met Asn Val Gly His Glu Asp Asp Glu Leu Lys
            340                 345                 350

Pro Tyr Val Glu Pro Leu Pro Asp Tyr Lys Asp Pro Arg Arg Thr Glu
        355                 360                 365

Leu Met Val Ser Met Gly Tyr Thr Arg Glu Glu Ile Gln Asp Ser Leu
    370                 375                 380

Val Gly Gln Arg Tyr Asn Glu Val Met Ala Thr Tyr Leu Leu Leu Gly
385                 390                 395                 400

Tyr Lys Ser

<210>  6

<211>  30

<212>  DNA

<213>  Artificial Sequence

<220>

<223>  oligonucleotide for PCR amplification and cloning

<220>

<221>  misc_feature

<222>  (1)..(30)

<223>  oligonucleotide derived from the 5' end of the TAO-1 sequence;  c
       ontains a NdeI-restriction site

<400>  6
tgactgctca gccatatgcc atcaactaac                                        30

<210>  7

<211>  33

<212>  DNA

<213>  Artificial Sequence

<220>

<223>  oligonucleotide for PCR amplification and cloning

<220>

<221>  misc_feature

<222>  (1)..(33)

<223>  oligonucleotide derived from the 3' end of the TAO-1 sequece; con
       tains a BamHI-restriction site

<400>  7
cagctccagc ggatccgcca ctttcaatta tta                                    33

<210>  8

<211>  107

<212>  DNA

<213>  Artificial Sequence

<220>

<223>  linker containing a N-terminal His10-Tag and an in-frame NdeI-res
       triction site for insertion into the XbaI-site of a modified pVL1
       392-Vector of Pharmingen

<220>

<221>  misc_feature

<222>  (1)..(107)

<223>  linker containing a N-terminal His10-Tag and an in-frame NdeI-res
       triction siite

<400>  8
tctagaaata attttgttta actttaagaa ggagatatac catgggccat catcatcatc      60

atcatcatca tcatcacagc agcggccata tcgaaggtcg tcatatg                   107